# FASCICULE DE BREVET EUROPEEN

(11) **EP 3 697 339 B1**
(45) Date de publication et mention de la délivrance du brevet: **10.07.2024**
(21) Numéro de dépôt: 18804670.0
(22) Date de dépôt: 28.09.2018
(51) Int. Cl.: A61F 2/04

(54) **DISPOSITIF CHIRURGICAL COMPLEXE POUR LA RÉALISATION ET PROTECTION D'UNE ANASTOMOSE**
KOMPLEXE CHIRURGISCHE VORRICHTUNG ZUR DURCHFÜHRUNG UND ZUM SCHUTZ EINER ANASTOMOSE
COMPLEX SURGICAL DEVICE FOR CARRYING OUT AND PROTECTING AN ANASTOMOSIS

(30) Priorité: 19.10.2017 FR 1759847
(43) Date de publication de la demande: 26.08.2020
(73) Titulaire: SafeHeal SAS, 75002 Paris (FR)
(72) Inventeur: KHOSROVANINEJAD, Charam, 84210 Pernes les Fontaines (FR); OSDOIT, Anne, 75009 Paris (FR); DUPONT, Cécile, 75018 Paris (FR)
(74) Mandataire: Vossius & Partner Patentanwälte Rechtsanwälte mbB
(86) Numéro de dépôt international: PCT/FR2018/052388
(87) Numéro de publication internationale: WO 2019/077218

(56) Documents cités:
- WO-A1-2008/030403
- WO-A1-2013/014353
- FR-A1- 2 846 868
- FR-A1- 2 941 858
- FR-B1- 2 846 868
- US-A1- 2007 032 879

## Description

La présente invention concerne un dispositif chirurgical complexe utile pour réaliser et protéger une anastomose intestinale.

Les anastomoses colorectales ont un taux de désunion avoisinant les 20%. La désunion d'une anastomose (fistule) est une complication grave avec une mortalité avoisinant les 20%. Pour diminuer les conséquences délétères des fistules anastomotiques certains patients bénéficient d'un abouchement (une stomie) ente la peau et l'intestin situé en amont de l'anastomose pour dériver le flux digestif dans une poche externe et éviter le contact entre l'anastomose et les matières fécales. La stomie est fermée dans un second temps après la cicatrisation de l'anastomose. La présence de stomie et la nécessité d'une réintervention pour la supprimer constituent des contraintes significatives pour le patient et une source de dépense de santé non négligeable.

C'est dans ce contexte qu'a été fourni un dispositif de protection de l'anastomose décrit dans FR 2 941 858 et EP2 395 942 qui permet de dériver les matières fécales dans la lumière d'intestin et d'éviter les stomies. Ce dispositif est composé d'une gaine externe souple solidarisée à un stent en aval de celui-ci, le dit stent étant destiné à être ancré en amont de l'anastomose. Le stent est l'élément d'ancrage temporaire aux parois de l'intestin, qui maintient la gaine en place et la gaine sert à dériver les matières fécales vers l'orifice anal sans contact avec la paroi intestinale au niveau de l'anastomose et ainsi protéger l'anastomose.

Dans WO 2013/014353, on a perfectionné ce dispositif en y ajoutant des moyens de contrôle de l'ancrage ou libération du stent par rapport à la paroi intestinale. Pour ce faire, le maintien en place ou la libération du dispositif sont renforcés au moyen d'un tube débouchant dans l'espace d'une chambre délimitée entre une gaine interne et la paroi du stent qui permet de créer par aspiration un effet de ventouse en attirant les parois de l'intestin contre le stent pour renforcer l'ancrage ou au contraire d'insuffler de l'air ou un fluide pour le libérer.

Pour ce faire, le dispositif chirurgical de WO2013/014353 consiste essentiellement à mettre à profit la viscoélasticité de l'intestin pour attirer sa paroi interne, vers la paroi externe du stent. En effet la muqueuse intestinale est souple et élastique tandis que les parois du stent sont relativement rigides. La paroi intestinale peut être attirée (par aspiration) et accolée aux parois externes du stent sous l'effet d'une pression négative dans l'interface stent-muqueuse. Dans ce cas, la force de friction entre l'intestin et le stent s'accroit rapidement et ce de façon significative de par une sorte d'effet ventouse. Par conséquent, la mobilité du stent devient étroitement liée à l'effet ventouse dont la modulation permet alors d'agir sur le comportement du stent dans l'intestin.

Plus précisément dans ce brevet WO 2013/014353 on décrit un dispositif chirurgical d'ancrage, apte à s'ancrer sur la muqueuse de la paroi interne de l'intestin comprenant ;
(a) un élément d'ancrage temporaire dont l'ancrage peut être modifié de façon contrôlée, comprenant au moins un premier élément longitudinal creux semi-rigide du type stent, définissant une paroi de révolution autour d'un axe longitudinal comprenant une partie courante multi-perforée sensiblement cylindrique à section sensiblement circulaire dite première paroi, ledit premier élément longitudinal creux étant réalisé en un matériau lui conférant des propriétés d'élasticité radiale de sorte qu'il peut être comprimé radialement en position rétractée et adopter une dite position d'expansion radiale maximale après relâchement de la compression radiale telle que ladite première paroi multi-perforée présente un premier diamètre externe que l'on peut faire varier de façon contrôlée entre :
   - un premier diamètre externe minimal en dite position radiale rétractée de ladite première paroi d'au plus 20 mm, de préférence d'au plus 10 mm, et
   - un premier diamètre externe maximal en dite position d'expansion radiale maximale de ladite première paroi, de préférence de 18 à 45 mm, et
(b) une gaine externe souple fixée au dit stent et s'étendant depuis son extrémité avale,
   caractérisé en ce qu'au moins une partie, de préférence, toute la longueur, de la surface interne cylindrique de ladite première paroi est doublée d'une couche étanche indépendante formant une gaine interne, seules les extrémités longitudinales de ladite gaine interne étant fixées de manière étanche audit élément d'ancrage à l'aide des premiers moyens de fixation étanche, de préférence par fusion (« joint fusing ») ou un joint annulaire de colle élastomère, à chaque dite extrémité longitudinale de ladite gaine interne, de façon à délimiter une chambre dénommée chambre de dépression entre ladite gaine interne et ladite première paroi, ledit élément d'ancrage temporaire étant couplé à un tube souple ou semi rigide dénommé tube d'injection-aspiration, s'étendant à l'extérieur dudit élément d'ancrage, une extrémité ouverte dudit tube d'injection-aspiration débouchant dans ladite chambre de dépression.

Plus particulièrement, ledit tube d'injection-aspiration est connecté, de préférence de façon réversible à son extrémité longitudinale libre à un embout de raccordement, lui-même connecté de façon réversible ou apte à être connecté de façon réversible avec un dispositif d'injection ou aspiration d'air ou liquide tel qu'une seringue, ledit embout de raccordement comprenant un dispositif d'obturation, de préférence une vanne anti-reflux et un dispositif de témoin de vide apte à indiquer la teneur du vide dans ladite chambre de dépression, notamment localisé sur une ampoule de vide.

Ladite chambre définit une chambre étanche entre le film étanche de la gaine interne et la paroi intestinale lorsque ledit élément d'ancrage est en position d'expansion maximale et immobilisé par ancrage contre la surface interne de la paroi intestinale. Et, en cas de décollement dudit élément d'ancrage et/ou en cas de migration dudit élément d'ancrage à l'intérieur de l'intestin, ladite chambre perd son étanchéité. Mais, on peut alors aspirer de l'air par ledit tube depuis son extrémité libre depuis l'extérieur du patient pour attirer par aspiration la paroi intestinale contre la surface externe de ladite première paroi sur une partie voire sur toute la surface externe de ladite première paroi délimitant ladite chambre pour stopper la migration de l'élément d'ancrage en rétablissant le vide dans ladite chambre et donc l'étanchéité de ladite chambre.

Ledit élément d'ancrage peut être maintenu en position radiale rétractée avec un instrument dénommé « introducteur » décrit ci- après, l'expansion radiale intervenant après dégagement de l'élément d'ancrage par rapport à l'introducteur.

Dans WO 2013/014353, l'introducteur peut être de façon connue constitué d'un tube guide semi rigide, du type cathéter muni à une de ses extrémités d'une poignée et dont le diamètre interne et la longueur permettent d'y maintenir logé ledit élément d'ancrage sous sa forme rétractée et ladite gaine, de préférence déployée longitudinalement.

Dans WO 2013/014353, pour une introduction par voie anale, ledit dispositif comprend en outre un instrument introducteur comprenant :
- une enveloppe externe tubulaire apte à contenir et maintenir ledit élément d'ancrage comprimé en dite position rétractée et ledit tube d'injection-aspiration à l'intérieur de l'extrémité distale de la dite enveloppe externe, et suffisamment long pour contenir en outre ladite gaine et ledit tube d'injection-aspiration, ladite enveloppe externe étant de préférence d'une longueur d'au moins 70 cm, de préférence au moins 100 cm, et
- des moyens pour acheminer l'extrémité distale dudit introducteur depuis l'orifice anal jusqu'au dit site d'ancrage dans l'intestin en amont de l'anastomose, et
- de préférence, des moyens pour dégager ledit élément d'ancrage par rapport à l'enveloppe externe, de préférence encore consistant en un tube butoir comportant une butée à son extrémité distale, en contact le cas échéant avec l'extrémité longitudinale dudit élément d'ancrage, ladite gaine en aval de l'élément d'ancrage entourant ledit tube butoir à l'intérieur de ladite enveloppe externe.

La réalisation de l'anastomose avec une agrafeuse se fait de manière connue avec une agrafeuse dite circulaire en introduisant une pièce à section transversale circulaire dénommée « enclume » dans l'intestin en amont du site de l'anastomose, au niveau de l'extrémité libre de l'intestin puis le serrage d'un fil dit « fil à bourse » autour de l'arbre de l'enclume avant d'engager le corps de l'agrafeuse dans la partie en aval de l'anastomose jusqu'au niveau de l'arbre de l'enclume pour faire coopérer l'agrafeuse circulaire avec l'enclume pour la section des tissus et la pose des agrafes.

Dans ces brevets antérieurs cités ci-dessus, le dispositif de protection de l'anastomose est donc placé dans l'intestin une fois l'anastomose réalisée avec une agrafeuse par exemple du type décrite dans FR 2 846 868. C'est grâce à un introducteur souple que le dispositif est introduit à travers l'orifice anal puis l'anastomose puis l'intestin situé en amont d'anastomose. Après retrait de l'introducteur, le stent s'ouvre en extension radiale, et vient au contact des parois d'intestin tandis que la gaine et le tube d'aspiration se déploient dans la lumière de l'intestin depuis son site d'ancrage en amont de l'anastomose jusqu'à l'orifice anal en traversant l'anastomose.

Malgré l'innocuité démontrée de ces manoeuvres certains chirurgiens demeurent réticents à l'idée d'introduire un objet à travers une anastomose fraîche comme décrit dans WO 2013/014355.

Selon la présente invention, on fournit un dispositif de protection que l'on peut introduire dans l'intestin avant même la confection de l'anastomose ceci aussi afin de faciliter la réalisation de l'anastomose avec l'enclume et l'agrafeuse, réduire le nombre d'étapes au cours de l'acte chirurgical et réduire le temps opératoire.

Pour ce faire, dans son principe la présente invention a pour objet :
- des moyens de mise en place du dispositif de protection d'anastomose dans la lumière de l'intestin en amont d'une anastomose ne nécessitant pas des instruments introduits par l'orifice anal et à travers l'anastomose fraîchement réalisée mais utilisant l'excision d'intestin pathologique et l'ouverture de la lumière d'intestin d'amont requise pour la mise en place de l'enclume de l'agrafeuse pour la mise en place préalable du dispositif de protection de l'anastomose, et
- des moyens de déploiement du dispositif d'ancrage, de la gaine externe et le cas échéant du tube d'aspiration à travers l'anastomose une fois celle-ci réalisée seulement, le déploiement de la gaine et du tube d'aspiration se faisant par et avec le retrait de l'agrafeuse, c'est-à-dire après les étapes de :
- fermeture de l'intestin d'amont sur l'axe de l'enclume au moyen d'un fil de bourse,
- introduction du corps d'agrafeuse à travers l'orifice anal dans l'intestin d'aval, et
- solidarisation de l'enclume avec le corps d'agrafeuse,
- agrafage, et
- retrait de l'enclume et l'agrafeuse à travers l'orifice anal.

Pour ce faire, la présente invention fournit plus précisément un dispositif chirurgical complexe constitué d'un ensemble prêt à être utilisé pour la réalisation d'une anastomose dans l'intestin avec une enclume et une agrafeuse circulaire puis la protection de ladite anastomose dans l'intestin comprenant :
(a) un dispositif de protection comprenant un élément d'ancrage constitué d'au moins un stent apte à s'ancrer temporairement sur la paroi interne de l'intestin en amont de l'anastomose et une gaine externe souple dont l'extrémité amont au moins est fixée audit stent, ladite gaine externe étant apte à s'étendre en aval dudit élément d'ancrage et en aval de la dite anastomose pour permettre la protection de la dite anastomose, notamment après réalisation de la dite anastomose et retrait des dites enclume et agrafeuse, et
(b) au moins un premier tube dénommé premier tube guide, le dit stent étant maintenu en compression radiale à l'intérieur du dit premier tube, de préférence au niveau d'une extrémité distale dudit premier tube guide, ledit premier tube étant une partie d'un tube guide d'un dispositif introducteur ou étant apte à être fixé de manière réversible à un dispositif introducteur, le dit premier tube étant déformable en incurvation par rapport à son axe longitudinal (XX'),
caractérisé en ce que ladite gaine souple est rangée, de préférence pliée, à l'intérieur du dit premier tube guide, et éventuellement en partie, voire entièrement, à l'intérieur dudit stent, l'extrémité avale de la dite gaine étant liée audit premier tube guide ou à une première pièce de raccordement indépendante dudit premier tube guide, ladite gaine souple étant apte à être déployée en aval dudit stent par le retrait de l'extrémité proximale dudit premier tube guide ou respectivement à ladite première pièce de raccordement à laquelle elle est fixée.

Plus particulièrement, l'extrémité proximale dudit premier tube guide ou respectivement ladite première pièce de raccordement comprend des moyens de fixation réversible tels que la dite extrémité proximale est apte à se fixer de manière réversible à une dite enclume, ladite gaine souple étant apte à être déployée en aval dudit stent par le retrait de la dite enclume fixée de manière réversible à l'extrémité proximale dudit premier tube guide ou respectivement à ladite première pièce de raccordement.

Plus particulièrement, l'extrémité proximale dudit premier tube guide ou respectivement ladite première pièce de raccordement comprennent des moyens de fixation réversible à la face frontale avant d'une dite enclume par collage, vissage, clampage ou liaison magnétique. On comprend que le retrait de la dite enclume implique la désolidarisation préalable de la dite enclume par rapport à la dite extrémité proximale dudit premier tube guide ou respectivement de ladite première pièce de raccordement.

De façon connue, un dit introducteur comprend au moins (i) ledit premier tube guide déformable, (ii) un deuxième tube guide rigide, l'extrémité proximale dudit premier tube guide déformable étant fixée ou apte à être fixée à l'extrémité distale dudit deuxième tube guide rigide, (iii) une poignée reliée à l'extrémité proximale dudit deuxième tube guide rigide, et (iv) un pousseur s'étendant depuis ladite poignée à l'intérieure dudit deuxième tube guide rigide et dudit premier tube guide déformable comprenant une tige de pousseur et une butée de pousseur à l'extrémité distale de la tige de pousseur.

De façon connue, ledit introducteur est apte à permettre l'implantation du dispositif de protection en amont du site de l'anastomose, la dite butée de pousseur étant apte à pousser le dit dispositif de protection à l'extérieur de l'extrémité distale dudit premier tube guide déformable pour permettre l'extension radiale et l'ancrage dudit stent contre la paroi de l'intestin, l'extrémité proximale de ladite tige de pousseur étant apte à coopérer avec ladite poignée en commandant manuellement une translation relative de ladite tige de pousseur par rapport au premier tube guide déformable.

Dans la technique antérieure, ce type d'introducteur est utilisé pour introduire le stent seulement après réalisation de l'anastomose et le dit premier tube guide déformable est plus long que selon l'invention car il doit s'étendre depuis l'orifice anal jusqu'en amont de l'anastomose, soit en pratique au moins 45 cm.

Selon la présente invention, l'introducteur est utilisé pour introduire le stent avant réalisation de l'anastomose de sorte que la longueur du dit premier tube guide déformable est par exemple de pas plus de 30 cm seulement, pour mettre en place le stent en amont du site de l'anastomose.

Ainsi le retrait de l'introducteur après ancrage du dit dispositif de protection en amont de l'anastomose permet de déployer partiellement la dite gaine dans l'intestin en aval de l'anastomose, le déploiement complet de la dite gaine externe se faisant lors du retrait de l'agrafeuse, via l'orifice anal. En effet, après le retrait des parties de l'introducteur autres que la dite première pièce de raccordement ou du dit premier tube de l'introducteur fixée à la dite gaine externe, ces dernières peuvent être fixées de manière réversible directement ou indirectement à une enclume d'agrafeuse en vue de réaliser l'anastomose comme explicité ci-après.

Plus particulièrement encore, lorsque l'extrémité avale de la dite gaine est fixée à une dite première pièce de raccordement, la dite première pièce de raccordement est positionnée juste en aval dudit stent, et les dits moyens de fixation réversible de ladite première pièce de raccordement, peuvent servir successivement à :
- 1) se fixer à une butée d'un pousseur dudit introducteur lors de la phase initiale d'introduction pour larguer ledit stent en dehors en amont dudit premier tube guide à l'issue de la phase initiale d'introduction sous l'effet de la poussée dudit pousseur, ledit stent se retrouvant ainsi dans l'intestin d'amont à une certaine distance de préférence de 10 à 20cm en amont du site d'anastomose, puis
- 2) se fixer à une dit enclume après détachement et retrait dudit pousseur et de l'ensemble dudit introducteur, pour permettre la réalisation de la dite anastomose avec une agrafeuse, éventuellement après déploiement partiel de la dite gaine en aval du stent par retrait dudit pousseur jusqu'au niveau du site de l'anastomose avant détachement dudit pousseur, le déploiement complet de la dite gaine en aval de ladite anastomose résultant du retrait de l'ensemble solidaire de la dite première pièces de raccordement, la dite enclume et dite agrafeuse après réalisation de la dite anastomose.

Plus particulièrement encore, lorsque l'extrémité avale de la dite gaine est liée audit premier tube, ledit premier tube est apte à être fixé de manière réversible à un dispositif introducteur, lesdits moyens de fixation réversible à l'extrémité proximale dudit premier tube guide servant successivement à :
- 1) se fixer à l'extrémité d'un deuxième tube guide de l'introducteur solidaire de la poignée et du pousseur, lors de la phase initiale d'introduction, puis
- 2) se fixer à la dite enclume, après détachement dudit premier tube guide et retrait du reste dudit introducteur incluant le retrait dudit pousseur, pour permettre la réalisation de la dite anastomose avec une enclume et une agrafeuse, éventuellement après déploiement partiel de la dite gaine en aval du stent en reculant dudit premier tube jusqu'au site de l'anastomose, le déploiement complet de la dite gaine en aval de ladite anastomose résultant du retrait complet de l'ensemble solidaire dudit premier tube guide, la dite enclume solidaire et la dite agrafeuse, après réalisation de la dite anastomose.

L'invention est plus particulièrement avantageuse lorsque le dispositif de protection comprend au moins un tube souple dénommé tube d'aspiration , de préférence deux tubes d'aspiration, chaque tube d'aspiration étant apte à s'étendre à l'extérieur dudit stent en aval de celui-ci, notamment après réalisation de la dite anastomose et retrait des dites enclume et agrafeuse, une extrémité ouverte dudit tube d'aspiration débouchant à l'intérieur du stent dans une chambre de dépression délimitée entre la paroi interne du stent et un film interne recouvrant la paroi interne du stent de préférence le dit film constituant une prolongation de la dite gaine, caractérisé en ce que chaque dit tube d'aspiration est rangé, de préférence plié, de préférence encore enroulé hélicoïdalement à l'intérieur du dit premier tube guide, et éventuellement en partie, voire entièrement, à l'intérieur dudit stent, l'extrémité avale de chaque dit tube d'aspiration étant liée audit premier tube guide ou à une dite première pièce de raccordement, chaque dit tube d'aspiration étant apte à être déployé en aval dudit stent par le retrait de la dite enclume fixée de manière réversible à l'extrémité proximale dudit premier tube guide ou respectivement à ladite première pièce de raccordement.

L'extrémité ouverte du tube d'aspiration consiste de préférence en une partie terminale du tube au sein de la dite chambre perforée par de multiples perforations.

La mise en oeuvre de deux tubes d'aspiration permet d'augmenter la force d'ancrage et protège contre une défaillance du premier tube d'aspiration, par exemple si ce dernier se bouche.

Plus particulièrement, un dispositif complexe prêt à être utilisé selon l'invention peut être mis en oeuvre dans un procédé de traitement chirurgical à l'aide d'un dispositif chirurgical complexe selon l'invention dans lequel on réalise les étapes successives suivantes dans lesquelles :
E1) on introduit le dit dispositif chirurgical dans l'intestin par voie abdominale dans la partie d'intestin en amont du site de l'anastomose avec un dispositif introducteur comprenant ou fixé à un dit premier tube guide et on largue le dit élément d'ancrage en amont du site de l'anastomose en actionnant un pousseur à l'intérieur dudit premier tube guide,
E2) on retire de l'intestin les parties du dit introducteur autre que le dit premier tube guide ou autre qu'une dite première pièce de raccordement auquel ou respectivement à laquelle sont fixés la dite gaine et ledit tube d'aspiration,
E3) on introduit une enclume d'agrafeuse que l'on fixe de manière réversible à l'extrémité proximale du dit premier tube guide ou à la dite première pièce de raccordement,
E4) on réalise la section des tissus de l'intestin de la zone de l'anastomose et la confection de l'anastomose par agrafage avec une dite agrafeuse coopérant avec la dite enclume,
E5) on retire l'agrafeuse fixée à l'enclume, elle-même fixée au dit premier tube ou à ladite première pièce de raccordement ce qui entraîne le déploiement de ladite gaine et du dit tube d'aspiration, et
E6) une fois traversé l'orifice anal, on désolidarise, de préférence avec des ciseaux, les extrémités avales de ladite gaine externe et du dit tube d'aspiration dudit premier tube guide ou de ladite première pièce de raccordement, à l'extérieur du dit orifice anal, et
E7) on raccorde l'extrémité avale du tube d'aspiration à un dispositif à l'extérieur du patient apte à maintenir le vide dans la chambre de dépression et ainsi empêcher la migration dudit élément d'ancrage.

Ce procédé est utile notamment pour la protection temporaire d'une anastomose sur le gros intestin ou côlon, le rectum ou canal anal, afin de prévenir ou réduire les risques de fistule anastomotique. Dans ce cas, de préférence, la distance entre ladite position d'ancrage en amont de ladite anastomose et ladite anastomose est au moins égale à 10 cm. Plus particulièrement, la distance entre l'anastomose et le site d'ancrage est d'au plus 20 cm.

Comme explicité plus loin, le dispositif selon l'invention du fait de sa possible implantation avant réalisation de l'anastomose permet en effet de mettre en oeuvre un stent et une gaine de dimensions réduites d'une part et donc implanté à une distance réduite en amont du site de l'anastomose par rapport à ce qui était requis dans les réalisations antérieures telles que dans WO 2013/014355, notamment à une distance de seulement 10 à 20cm seulement.

Par « protection de l'anastomose », on entend ici une protection de celle-ci lors de la reprise du transit intestinal.

Par « aval » et « amont », on se réfère ici au sens de cheminement d'amont en aval du transit intestinal et à une position lorsque les dispositifs concernés sont en place dans l'intestin pour remplir leur fonction. Par « extrémité avale » de ladite gaine, on comprend qu'il s'agit de l'extrémité destinée à se déployer en au dudit stent une fois déployée après retrait de l'agrafeuse après réalisation de l'anastomose, même si cette extrémité avale est initialement rapprochée de l'extrémité amont pour être fixée à la paroi interne du premier tube guide.

Par « proximal » et « distal », on se réfère ici à la position dans ou de l'élément concerné par rapport au site d'introduction initial dans le corps du patient et en pratique ici par rapport aussi à la poignée de l'introducteur s'agissant des éléments de l'introducteur. Les extrémités proximale et amont, correspondent aux extrémités avale et amont respectivement pour une introduction par l'orifice anal ou une partie d'intestin en aval de l'anastomose.

En l'espèce selon l'invention, seul le premier tube guide déformable de l'introducteur et une partie du dit deuxième tube guide rigide sont introduits dans la lumière de la partie de l'intestin en amont de l'anastomose, le reste du deuxième tube guide rigide et la poignée restant en dehors de l'intestin.

Par « premier tube guide déformable » de l'introducteur, on entend en pratique un tube semi flexible en matériau élastomère ou PU plus épais que la dite gaine et déformable par incurvation par rapport à son axe longitudinal jusqu'à pouvoir adopter une courbure à 90°externe. Ce premier tube guide déformable présente des graduations permettant de contrôler le site d'implantation avant largage.

Plus particulièrement, de façon connue, ladite gaine est fixée de manière étanche audit élément d'ancrage à l'aide de moyens de fixation étanche, de préférence par joint de fusion (« fusing joint ») ou un joint annulaire de colle élastomère, à chaque dite extrémité longitudinale de ladite gaine interne, de façon à délimiter une chambre de dépression entre ladite gaine interne et ladite première paroi, ledit élément d'ancrage temporaire étant couplé au dit tube souple ou semi rigide dénommé tube d'aspiration, s'étendant à l'extérieur dudit élément d'ancrage, une extrémité ouverte dudit tube d'aspiration débouchant dans ladite chambre de dépression.

Dans un premier mode de réalisation l'extrémité avale de la dite gaine et l'extrémité avale du (ou des) dit(s) tube(s) d'aspiration, sont reliées à l'extrémité distale du dit premier tube guide, l'extrémité proximale (ou extrémité avale) dudit premier tube guide comprenant une deuxième pièce de raccordement fixée ou apte à se fixer de manière réversible à l'extrémité distale (ou extrémité amont) d'un deuxième tube guide rigide d'un dispositif introducteur pour l'introduction du dispositif de protection en amont du site de l'anastomose et ensuite apte à se fixer de manière réversible à une dite enclume pour la réalisation de l'anastomose.

La deuxième pièce de raccordement est fixée ou apte à fixer de manière réversible le dit premier tube guide à l'extrémité distale d'un deuxième tube guide rigide d'un dispositif introducteur pour les solidariser pour l'introduction du dispositif de protection en amont du site de l'anastomose puis les désolidariser pour le retrait de l'introducteur avant la mise en oeuvre de l'enclume et l'agrafeuse.

Dans ce premier mode de résiliation, ledit premier tube guide comprenant une deuxième pièce de raccordement à son extrémité proximale reste introduit dans l'intestin pendant la réalisation de l'anastomose et est retiré de l'intestin lors du retrait de l'agrafeuse après la réalisation de l'anastomose du fait de sa liaison avec l'enclume permettant ainsi le dépliement et déploiement complet de la dite gaine et du dit tube d'aspiration en aval du dit stent du fait de leur liaison avec le dit premier tube.

Plus particulièrement, dans ce premier mode de réalisation, l'extrémité avale de la dite gaine et l'extrémité avale du (ou des) dit(s) tube(s) d'aspiration sont reliées à l'extrémité distale (ou extrémité amont) du dit premier tube guide par collage ou par un fil.

Dans ce premier mode de réalisation, plus particulièrement, la gaine et le (ou les) tube(s) d'aspiration sont entièrement rangés, notamment pliés ou enroulés, à l'intérieur du stent excepté leurs parties avales qui sortent du stent à son extrémité avale pour rejoindre l'extrémité amont du premier tube guide en amont du stent, ces parties externes de la gaine et du tube d'aspiration étant intercalées entre le stent et la paroi interne du premier tube guide.

Plus particulièrement, dans ce premier mode de réalisation, la dite deuxième pièce de raccordement est fixée ou apte à se fixer de manière réversible à un adaptateur lui-même apte à se fixer de manière réversible à une dite enclume d'agrafeuse.

Plus particulièrement encore, dans ce premier mode de réalisation, ladite deuxième pièce de raccordement est fixée ou apte à se fixer de manière réversible par vissage, collage, clampage ou liaison magnétique, à un adaptateur lui-même apte à se fixer de manière réversible à une dite enclume d'agrafeuse, par collage, clampage ou liaison magnétique.

Plus particulièrement encore, dans ce premier mode de réalisation, ladite deuxième pièce de raccordement comprend un élément de filetage apte à coopérer par vissage avec :
- un premier élément de filetage complémentaire situé à l'extrémité distale du dit deuxième tube guide rigide, puis
- un deuxième élément de filetage complémentaire situé à l'extrémité distale d'un dit adaptateur.

On comprend que :
- ladite deuxième pièce de raccordement est fixée de manière réversible à l'extrémité distale du dit deuxième tube guide rigide, lors de l'introduction et largage du dispositif de protection par le dit pousseur pour permettre l'introduction puis l'ancrage dudit stent dans l'intestin en amont du site de la dite anastomose avant réalisation de l'anastomose, et
- ladite deuxième pièce de raccordement est fixée de manière réversible à la dite enclume ou un dit adaptateur après retrait dudit pousseur en vue de la fixation réversible de l'enclume pour permettre la réalisation de la dite anastomose avec la dite agrafeuse après que le dit stent ait été ancré dans l'intestin en amont du site de la dite anastomose.

Plus particulièrement, la partie proximale plate d'un dit adaptateur est apte à se fixer par collage à la face frontale plate d'une dite enclume.

Dans un deuxième mode de réalisation, l'extrémité avale de la dite gaine et de préférence l'extrémité avale du (ou des) dit(s) tube(s) d'aspiration, est (ou sont) liée(s) à une dite première pièce de raccordement dont une extrémité proximale au moins est disposée à l'extérieure de l'extrémité avale dudit stent.

Plus particulièrement, dans ce deuxième mode de réalisation, l'extrémité avale de la dite gaine et de préférence l'extrémité avale du (ou des) dit(s) tube(s) d'aspiration est (ou sont) liée(s) à une partie distale de la dite première pièce de raccordement indépendante dudit premier tube guide, de préférence une partie distale tubulaire rangée ou apte à se ranger à l'intérieur de l'extrémité avale dudit stent, et une partie proximale de la dite première pièce de raccordement est disposée à l'extérieur et à l'extrémité avale dudit stent présentant, de préférence une face proximale plate de plus grand diamètre que ladite partie distale tubulaire.

Ainsi, initialement et lors de la phase d'introduction, ladite première pièce de raccordement est disposée à l'intérieur dudit premier tube guide.

Dans ce deuxième mode de réalisation, plus particulièrement, la gaine et le tube d'aspiration sont entièrement rangés à l'intérieur du stent excepté leurs parties avales qui sortent du stent à son extrémité avale pour rejoindre la première pièce de raccordement plaquées contre l'extrémité avale du stent.

Dans ce deuxième mode de réalisation, l'ensemble du dispositif de protection et de la dite première pièce de raccordement sont indépendants du premier tube guide et de l'introducteur.

Plus particulièrement, l'extrémité avale de la dite gaine et l'extrémité avale du (ou des) dit(s) tube(s) d'aspiration, sont liées à une dite première pièce de raccordement par collage ou par un fil.

Plus particulièrement encore, dans ce deuxième mode de réalisation la dite première pièce de raccordement est apte à se fixer de manière réversible à une butée d'une tige de pousseur à l'intérieur dudit premier tube guide lors de l'introduction du dispositif de protection en amont du site de l'anastomose, de préférence par collage, vissage, clampage ou liaison magnétique, de préférence encore par collage.

Plus particulièrement encore, dans ce deuxième mode de réalisation la dite première pièce de raccordement est apte à se fixer de manière réversible directement à une dite enclume par collage, vissage, clampage ou liaison magnétique de préférence par collage.

Dans ce mode de résiliation, le dispositif introducteur peut être entièrement retiré de l'intestin après introduction du dispositif de protection en amont du site de l'anastomose avant réalisation de l'anastomose, seule la dite première pièce de raccordement restant introduite dans l'intestin pour être fixée à l'enclume en vue de la réalisation de l'anastomose, la dite première pièce de raccordement étant retirée de l'intestin lors du retrait de l'agrafeuse après la réalisation de l'anastomose du fait de sa liaison avec l'enclume, permettant ainsi le déploiement complet de la dite gaine et du (ou des) dit(s) tube(s) d'aspiration en aval du dit stent du fait de leur liaison avec la dite première pièce de raccordement.

On comprend que :
- la dite première pièce de raccordement est fixée de manière réversible à ladite butée du pousseur lors de l'introduction et largage du dispositif de protection par le dit pousseur pour permettre l'introduction puis l'ancrage dudit stent dans l'intestin en amont du site de la dite anastomose avant réalisation de l'anastomose, et
- la dite première pièce de raccordement est fixée de manière réversible à ladite enclume d'agrafeuse ou à un dit adaptateur après retrait complet dudit introducteur pour permettre la réalisation de la dite anastomose avec la dite agrafeuse après que le dit stent ait été ancré dans l'intestin en amont du site de la dite anastomose.

Plus particulièrement encore, pour les deux mode de réalisation l'extrémité distale du dit premier tube guide est fermée par un élément de retenue flexible apte à retenir le dit dispositif de protection à l'intérieur du dit premier tube guide en absence de poussée par une butée de tige de pousseur, élément de retenue étant apte à se déformer élastiquement et autoriser la sortie dudit dispositif de protection sous l'effet de la poussée par la dite butée du pousseur.

Plus particulièrement encore, le dispositif chirurgical complexe selon l'invention comprend un dit dispositif de protection et un introducteur apte à permettre l'implantation du dispositif de protection en amont du site de l'anastomose, ledit introducteur comprenant une poignée fixée et/ou apte à coopérer avec les deux parties suivantes de l'introducteur :
(b1) un premier tube guide déformable à l'intérieur duquel ledit stent est maintenu en compression radiale à proximité de l'extrémité distale dudit premier tube guide déformable, l'extrémité proximale dudit premier tube guide déformable étant fixée à un deuxième tube guide rigide solidaire de la dite poignée, et
(b2) un pousseur comprenant une tige de pousseur déformable en incurvation par rapport à son axe longitudinal et une dite butée de pousseur à l'extrémité distale de la tige de pousseur s'étendant depuis la poignée à l'intérieure dudit deuxième tube guide rigide et dudit premier tube guide déformable, l'extrémité proximale de ladite tige de pousseur étant apte à coopérer avec ladite poignée en commandant manuellement une translation relative de ladite tige de pousseur par rapport au premier tube guide déformable.

Plus particulièrement, la tige de pousseur est une tige spiralée formée d'un fil en acier enroulé hélicoïdalement selon un axe longitudinal virtuel XX' avec des spires jointives coaxiales de même diamètre, le diamètre des spires jointives formant une tige déformable apte à être déformée en incurvation par rapport audit axe longitudinal pour permettre d'adopter une courbure jusqu'à former un coude à 90°.

Plus particulièrement encore, ledit stent comprend au moins une boucle de retrait à une extrémité de ses extrémités longitudinales, de préférence deux boucles à respectivement chacune de ses extrémités longitudinales.

Les caractéristiques avantageuses et originales du procédé d'ancrage du stent et réalisation de l'anastomose découlant des caractéristiques structurelles originales du dispositif chirurgical complexe selon l'invention sont :
- un ancrage du dispositif de protection en amont du site d'anastomose, avec déploiement de ladite gaine et du dit tube d'aspiration et retrait de l'agrafeuse en un seul geste après réalisation d'une anastomose par agrafeuse chirurgicale,
- absence de passage de l'introducteur via l'anastomose fraîche, et
- réduction du temps opératoire.

Plus particulièrement, le tube d'aspiration est un tube semi rigide notamment en PE ou PP et présente une longueur apte à tenir en forme et s'étendre à l'intérieur de l'intestin depuis un orifice anal du patient jusqu'au dit élément d'ancrage, de préférence la longueur dudit tube d'injection-aspiration étant au moins de 20 cm, plus particulièrement de 50 à 150 cm, et l'extrémité libre dudit tube d'aspiration à l'extérieur du patient est raccordée à un dispositif d'aspiration ou injection de fluide gazeux ou liquide, notamment de l'air ou un liquide froid comme explicité ci-après.

Plus particulièrement, la longueur de la gaine externe protège l'anastomose, et dépasse de l'orifice anal lorsque ledit élément d'ancrage est en position d'ancrage et qu'elle est déployée en aval de celui-ci.

Ladite gaine externe de par sa constitution en élastomère présente des propriétés d'étirabilité radiale et longitudinale similaires à celle de la paroi intestinale, propriétés qui sont celles d'un matériau élastomère en forme de dite gaine, celui-ci présentant des propriétés d'élasticité radiale et longitudinale. Ces propriétés d'élasticité radiale et longitudinale de la gaine sont similaires à celles de la paroi du côlon.

Dans un mode préféré de réalisation, ladite partie courante de dite première paroi s'étend depuis l'extrémité longitudinale amont de ladite première paroi jusqu'à une partie terminale avale profilée de diamètre plus petit que celui de la partie courante cylindrique, le diamètre externe en expansion radiale maximale de l'extrémité avale de ladite première paroi étant de 20 à 40 mm, et la longueur de ladite partie terminale profilée de la première paroi étant de 10 à 30 mm, de préférence de 15 à 25 mm, de préférence le diamètre de ladite partie terminale diminuant progressivement entre ladite partie courante et ladite extrémité avale de ladite première paroi.

Plus particulièrement, ledit élément d'ancrage temporaire est une prothèse entérale dont ladite première paroi est formée par un maillage de fils spiralés de préférence des fils métalliques. De façon connue, l'expansion radiale résulte alors du croisement des fils métalliques dont la variation angulaire permet de varier la largeur du losange ou parallélogramme des mailles dudit maillage de fils spiralés.

Avantageusement, ledit élément d'ancrage est réalisé en un matériau qui lui confère une dite expansion par élasticité radiale uniquement à température au moins égale à la température ambiante de 20°C, notamment à la température du corps humain, ledit élément d'ancrage étant en dite position radiale rétractée à une température inférieure à ladite température ambiante, de préférence inférieure à 5°C. On comprend que le matériau en forme tubulaire change de diamètre automatiquement en fonction de la température ambiante.

Plus particulièrement encore, ledit élément d'ancrage est une prothèse entérale dont ladite première paroi est formée par un maillage de fils spiralés, de préférence en nitinol. Le nitinol est un alliage métallique présentant des propriétés d'expansion radiale progressive en fonction de la température, à température supérieure ou égale à la température ambiante (25°C), ce qui permet de le conserver sous forme rétractée à température plus froide, notamment à 4°C au stockage. Une fois rétracté à basse température, il reste ainsi rétracté suffisamment de temps pour pouvoir le loger dans le tube introducteur et l'acheminer dans l'intestin à l'aide dudit introducteur. Une fois libérée dans l'intestin, la prothèse retrouve progressivement son expansion radiale sous l'effet d'une température ambiante plus élevée, celle du corps humain. En effet, pour les stents en nitinol, la mémoire de forme de cet alliage permet de modifier sa forme et sa rigidité en fonction de la température ambiante. Plus particulièrement, aux températures situées en dessous de 15°, le nitinol devient souple et malléable. Ainsi, l'injection d'une solution liquide froide entre 0° et 15° dans la chambre de dépression permet de rendre malléable le stent et de faciliter sa mobilisation lors du passage à travers l'anastomose ou d'une zone de rétrécissement par exemple.

Avantageusement, ladite gaine est en matériau synthétique biocompatible, d'épaisseur de paroi de 0,01 à 1 mm, de préférence en matériaux élastomère du type silicone ou polyuréthane de 0,05 à 1 mm d'épaisseur, et de préférence encore présentant des propriétés d'élasticité radiale et longitudinale, et ladite gaine externe au moins présentant des propriétés de mémoire de forme et des propriétés de non collabilité.

On comprend que ladite gaine externe de par sa constitution en élastomère présente des propriétés d'étirabilité radiale et longitudinale similaires à celle de la paroi intestinale, propriétés qui sont celles d'un matériau élastomère en forme de dite gaine externe, celui-ci présentant des propriétés d'élasticité radiale et longitudinale. Ces propriétés d'élasticité radiale et longitudinale de la gaine externe sont similaires à celles de la paroi du côlon et permettent que le transit intestinal se fasse correctement dans ladite gaine externe pendant toute la durée de migration de l'élément d'ancrage soit une durée d'au moins 6 à 10 jours.

L'élasticité longitudinale de la gaine externe élastomère peut être plus importante que celle de l'intestin, sans que cela ne pose de difficulté, au contraire elle présente l'avantage de pouvoir être tirée sur la partie de gaine externe en dépassement anal, de manière à la couper et à ce qu'elle se rétracte à l'intérieur en amont dans le rectum. De par son élasticité radiale, ladite extrémité longitudinale de la gaine externe reste fixée à ladite extrémité dudit élément d'ancrage quel que soit son niveau d'expansion radiale.

D'autre part, les caractéristiques d'épaisseur de la gaine externe combinée à son élasticité lui confèrent une propriété de mémoire de forme. On entend ici par « propriétés de mémoire de forme » que le matériau élastomère constitutif de ladite gaine externe retrouve naturellement sa forme initiale lorsqu'il est déformé par pliage. Comptetenu de la grande longueur de la gaine externe, ces propriétés de mémoire de forme sont importantes, pour que le matériau retrouve naturellement sa forme longitudinale sans créer de blocage du transit en cas de plicatures de la gaine externe lesquelles peuvent effectivement intervenir pendant sa migration après libération de l'élément d'ancrage.

On entend par « propriétés de non collabilité » que le matériau élastomère constitutif de ladite gaine externe présente un coefficient d'adhérence tel que les deux surfaces opposées de paroi interne de la gaine externe ne se collent pas l'une contre l'autre lors de pliement, ceci pour ne créer aucune résistance au passage des gaz et matières.

On comprend aussi que :
- ladite gaine externe présente un diamètre au repos sensiblement au moins égal au dit diamètre externe réduit en rétractation radiale dudit élément d'ancrage creux, et inférieur à celui de l'intestin au repos, de préférence ledit diamètre au repos de ladite gaine externe est sensiblement égal à celui de la paroi intestinale au repos, et
- ladite gaine externe s'étend en aval de l'extrémité dudit élément d'ancrage à laquelle elle est fixée sur une longueur correspondant à la distance entre la position d'ancrage en amont de ladite anastomose et une position en aval de préférence jusqu'à l'orifice anal.

De façon connue, l'expansion radiale résulte alors du croisement des fils métalliques dont la variation angulaire permet de varier la largeur du losange ou parallélogramme des mailles dudit maillage de fils spiralés.

De préférence, et de façon connue, on utilise un stent dont le dessin et la forme du maillage des fils spiralés permettent d'obtenir une variation du diamètre dudit stent avec un minimum de variation de longueur, de préférence pratiquement sans variation de longueur à la compression radiale.

D'autres caractéristiques et avantages de la présente invention ressortiront mieux à la lecture de la description qui va suivre, faite de manière illustrative et non limitative, en référence aux dessins annexés suivants.

Les figures 1A, 1B et 1C représentent des vues schématiques d'un dispositif de protection 5 selon l'invention comprenant une vue de côté avec la gaine 2 et tube d'aspiration 3 déployés en aval du stent 1 (figure 1A), une vue frontale de l'extrémité amont ou extrémité distale du stent 1 montant une boucle lasso 6b (figure 1B) et une vue en coupe longitudinale du dispositif de protection 5 montrant les positions respectives du stent 1, de la gaine 2 et du tube d'aspiration 3, ce dernier débouchant dans une chambre 4 entre la paroi du stent et la partie interne 2a de la gaine 2.

La figure 2 montre les différentes parties d'un dispositif introducteur 10 démonté.

La figure 2A représente le dispositif introducteur 10 avec le premier tube guide 12 introduit à l'intérieur de la partie de l'intestin d'amont 100a.

La figure 2B représente le largage du stent en dehors du premier tube guide 12 par la poussée de la tige du pousseur 15 (non visible sur la figure 2B).

Les figures 3A et 3B représentent le détail d'une tige de pousseur déformable 15b de forme spiralée en vue de côté (figure 3A) et en vue frontale d'une de ses extrémités (figure 3B).

Les figures 4A à 4C représentent différentes étapes de la mise en place d'une enclume 8 et de la réalisation de l'agrafage d'une anastomose 101 entre la partie d'amont 100a et la partie d'aval 100b d'intestin avec une agrafeuse 9 coopérant avec une enclume 8.

Les figures 5A-5B, 6 et 7A-7D concernent le mode de réalisation d'exemple 1.

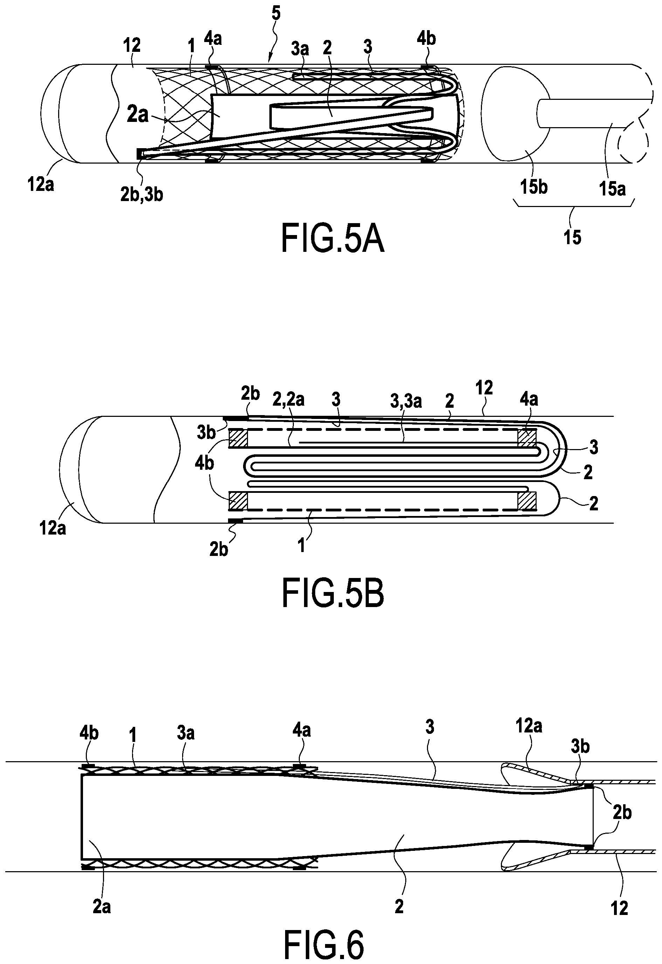

Les figures 5A et 5B représentent deux variantes en vues schématiques en coupes (partiellement éclatée pour la figure 5A) d'un dispositif de protection 5 avec le stent comprimé à l'extrémité distale du premier tube 12 dans lequel la gaine 2 et le tube d'aspiration 3 sont pliés à l'intérieur du stent, leurs extrémités avales étant collées à l'extrémité distale du premier tube, le dispositif de protection 5 étant prêt à être expulsé par la butée 15b du pousseur 15 en dehors du premier tube guide 12.

La figure 6 montre d'une manière schématique le stent 1 largué à l'extérieur avec le premier tube guide 12 séparé du reste de l'introducteur et vidé de la tige pousseur après largage du stent et le déploiement de la gaine 2 et du tube d'aspiration 3 en aval du stent 1 du fait du retrait du premier tube guide 12 auxquels ils sont liés.

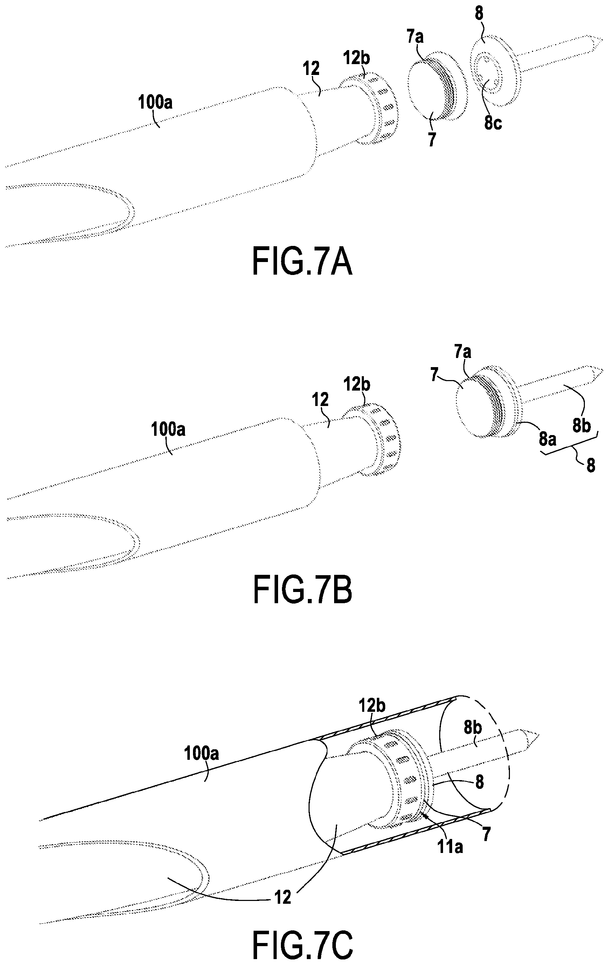

Les figures 7A à 7C représentent différentes étapes de mise en oeuvre de l'enclume à l'extrémité proximale du premier tube guide 12 avant réalisation de l'anastomose.

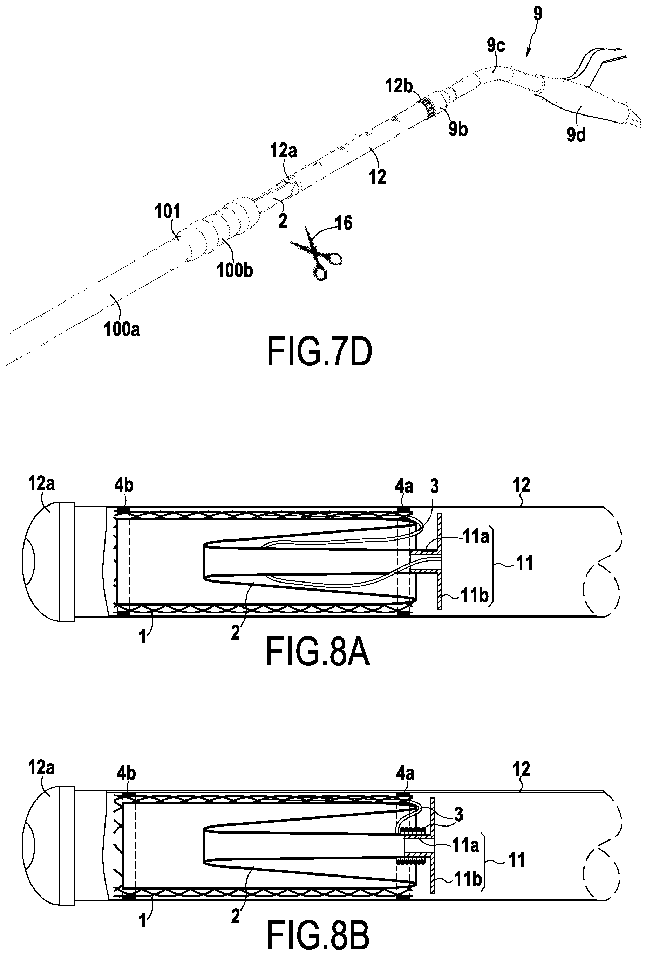

La figure 7D représente le retrait de l'agrafeuse solidaire du premier tube 12 permettant le déploiement de la gaine 2 après réalisation de l'anastomose.

Les figures 8A-8B, 9A-9B, 10A-10C et 11A-11B représentent le mode de réalisation de l'exemple 2.

Les figures 8A et 8B représentent 2 variantes de fixation du tube d'aspiration 3 et de la gaine 2 sur une première pièce de raccordement 11 en aval du stent 1 et indépendante du premier tube guide.

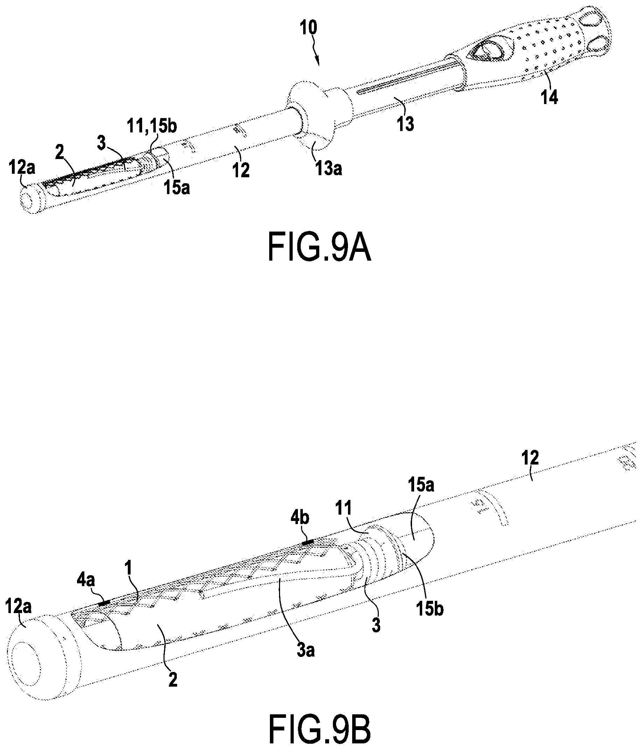

Les figures 9A et 9b montrent un dispositif de protection 5 avec le stent 1 à l'extrémité distale du premier tube guide 12 et la gaine 2 et le tube d'aspiration 3 fixés à un adaptateur 11 lui-même fixé de manière réversible à la butée 15b d'un pousseur 15.

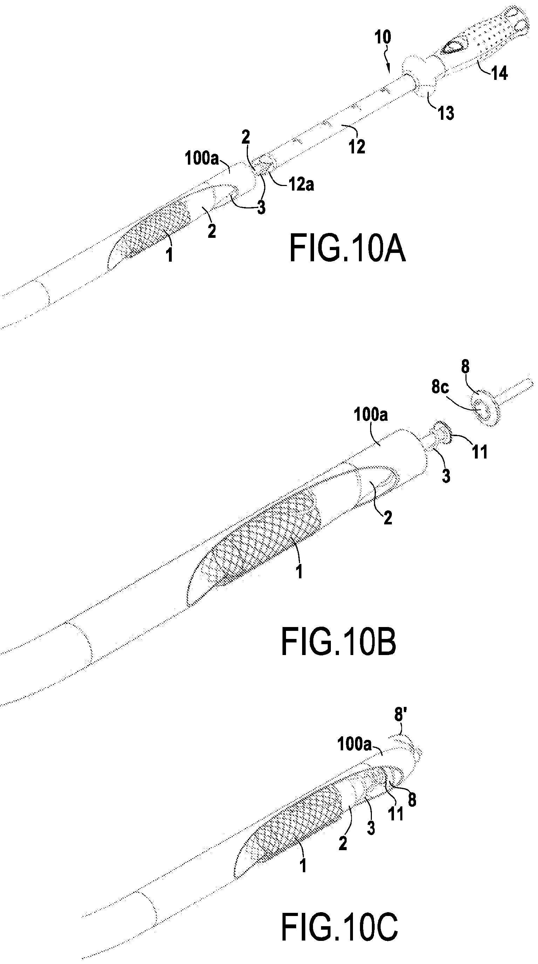

Les figures 10A, 10B et 10C représentent différentes étapes du retrait de l'introducteur 10 y compris le premier tube guide 12 après largage du stent 1 et déploiement partiel de la gaine 2 et du tube d'aspiration 3 liés à la première pièce de raccordement 11.La figure 10B montre l'approche de l'enclume 8 en vis-à-vis de la première pièce de raccordement 11.La figure 10C montre la fixation de l'enclume 8 dans l'intestin d'amont 10a avant réalisation de l'anastomose avec l'agrafeuse 9.

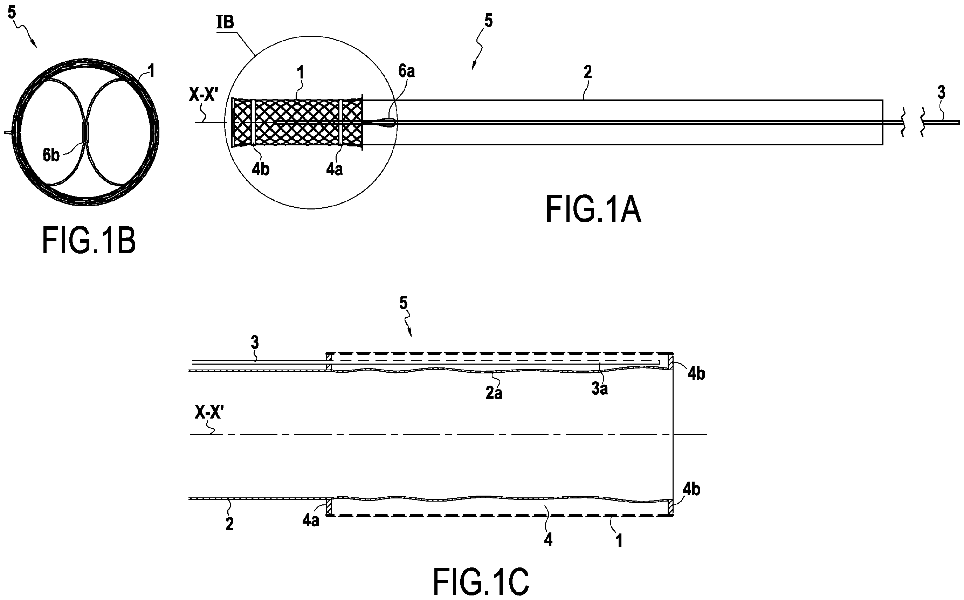

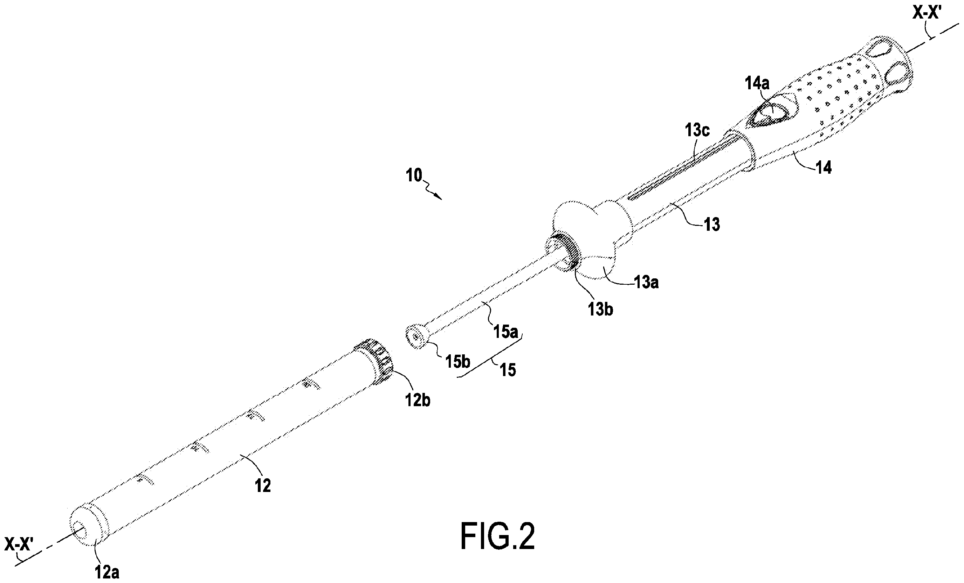

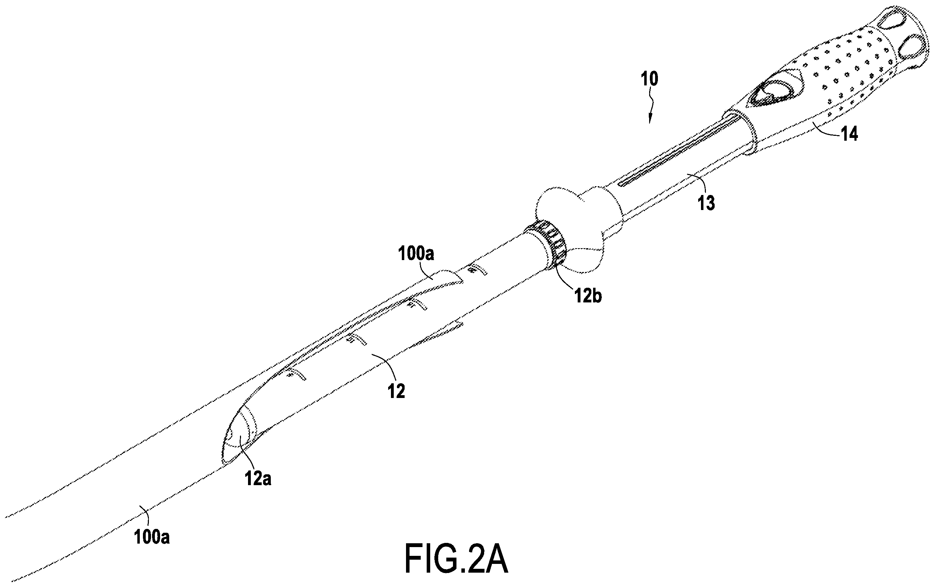

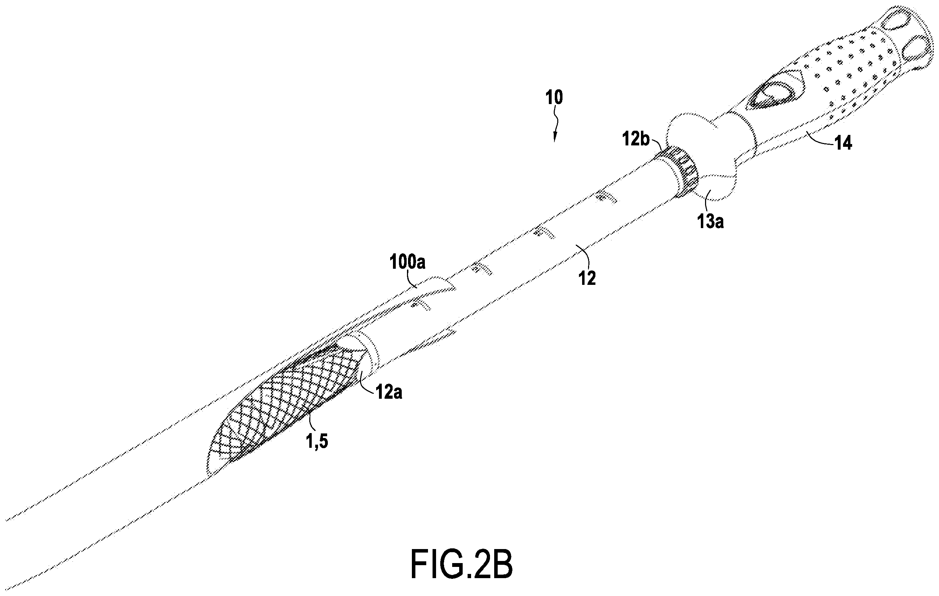

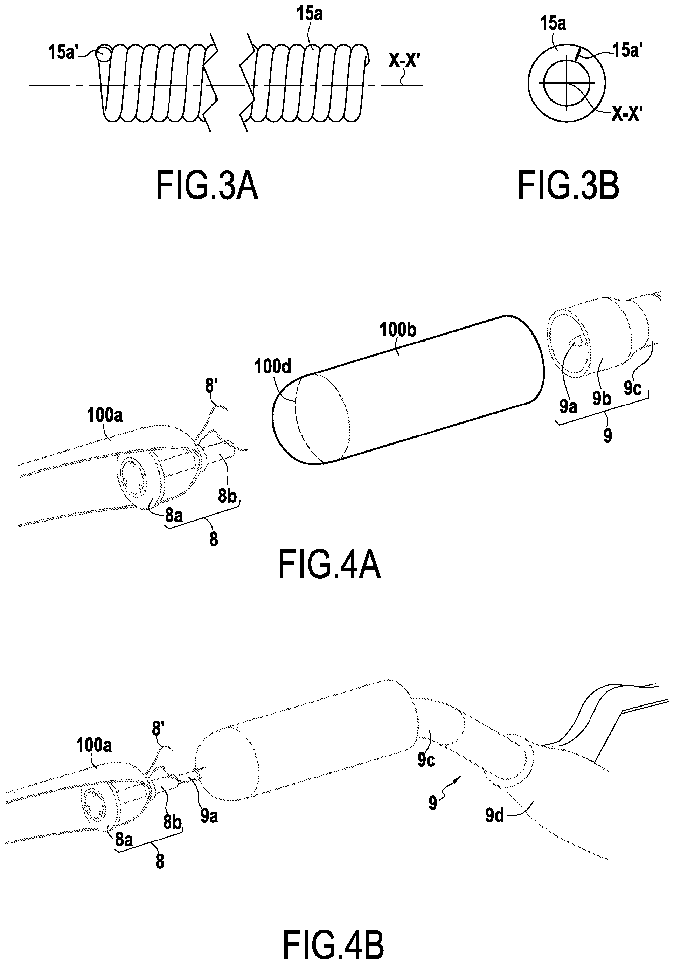

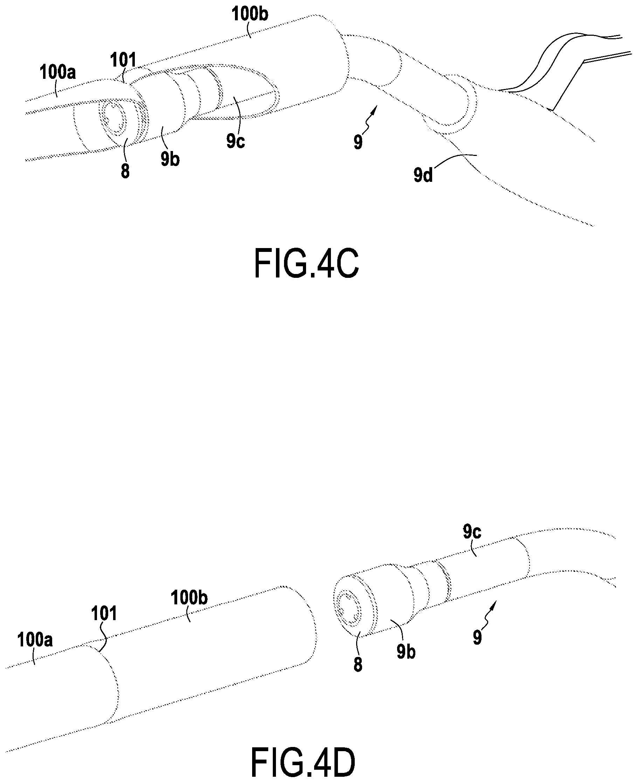

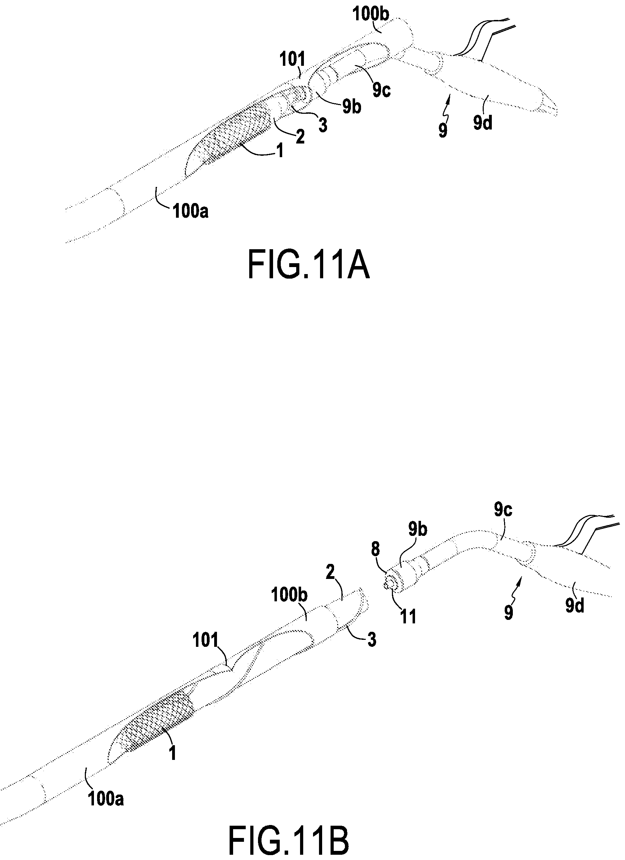

Les figures 11A et 11B représentent l'agrafeuse 9 avec l'enclume 8 solidaires des extrémités avales de la gaine 2 et du tube 3 lors de la réalisation de l'anastomose 101 (figue 11A) et après retrait de l'agrafeuse et séparation d'avec la première pièce de raccordement 11 après réalisation de l'anastomose 101 (figure 11B).Dans les deux modes de réalisation des exemples 1 et 2 ci-après, on met en oeuvre un dispositif de protection d'anastomose 5 des figures 1A-1Cdu type décrit dans WO2013/014353 conditionné dans une partie d'un dispositif introducteur tel que décrit ci-après en référence aux figures 2, 2A-2B et 3A-3B en vue de la réalisation d'une anastomose décrite en référence aux figures 4A-4C.

Le dispositif de protection d'anastomose 5 comprend un élément d'ancrage constitué par un stent 1 dont la paroi interne est recouverte d'une gaine interne 2a souple délimitant une chambre annulaire 4 entre des joints annulaires 4a-4b étanche entre la gaine interne 2a et la paroi perforée du stent. La gaine interne 2a constitue un film étanche formant une paroi tubulaire souple et se prolonge d'une gaine externe souple 2 s'étendant à l'extérieur dudit élément d'ancrage dans la direction longitudinale dudit stent. Toute la longueur de la surface interne cylindrique de la paroi interne du stent est donc doublée d'une couche étanche indépendante formant une gaine interne 2a, seules les extrémités longitudinales de ladite gaine interne 2a étant fixées de manière étanche audit élément d'ancrage 1 à l'aide des joints annulaires élastomère obtenu par fusion à chaque dite extrémité longitudinale de ladite gaine interne.

De façon connue, le stent 1 comprend avantageusement à ses extrémités amont et aval des collerettes évasées comme représentées dans le brevet WO 2013/014353.

On comprend que la gaine interne 2a n'est pas tendue d'une manière excessive pour ne pas rigidifier le stent, de sorte que l'écart entre ladite gaine interne 2a et le diamètre externe maximal du stent est de préférence de 0,2 à 10 mm, de préférence encore de 1 à 5 mm, et l'espace entre ladite gaine interne 2a et ladite paroi du stent définit une chambre dénommée chambre de dépression 4.

Le dispositif de protection 5 selon l'invention comprend en outre un tube souple ou semi-rigide dénommé tube d'aspiration 3 s'étendant à l'extérieur en aval dudit stent et débouchant dans la chambre de dépression 4 entre la gaine interne 2a et la paroi du stent 1. Une partie amont du tube d'aspiration 3 comprenant de multiples perforations, s'étend sensiblement sur toute la longueur de ladite chambre 4 dans la direction longitudinale XX du dispositif. Le tube d'aspiration 3 débouche dans ladite chambre de dépression en traversant de façon étanche le joint annulaire élastomère à l'extrémité avale de la gaine interne 2a lorsque le tube 3 est introduit par l'orifice anal. Ledit tube d'aspiration 3 et ladite gaine externe 2 s'étendent à l'extérieur dudit stent depuis la même extrémité avale dudit stent.

Le tube d'aspiration 3 sert à injecter ou aspirer de l'air dans la chambre 4 pour aspirer la paroi intestinale 100a contre la face externe du stent 1, et plus généralement pour modifier la caractéristique d'ancrage du stent 1 par rapport à la paroi intestinale 100a.

La portion 3a du tube aspiration 3 à l'intérieur de la chambre 4 peut être collée sur la face interne du stent 1 ou sur la face externe de ladite gaine externe 2.

En variante, la gaine externe 2 peut être fixée à son extrémité amont au même joint de fusion en élastomère 4a-4b ou peut être fixée sur la face externe de l'extrémité longitudinale avale du stent par recouvrement de celle-ci sur une courte portion de longueur (non représenté sur les figures).

Le stent du type réalisé par maillage spiralé de fils métalliques en nitinol lui conférant des propriétés d'élasticité radiale de sorte qu'il peut être comprimé radialement en position rétractée et adopter une dite position d'expansion radiale maximale après relâchement de la compression radiale telle que ladite première paroi multi-perforée présente un premier diamètre externe que l'on peut faire varier de façon contrôlée entre :
- un diamètre externe minimal en dite position radiale rétractée de ladite première paroi, de 16 mm, et
- un diamètre externe maximal en dite position d'expansion radiale maximale de ladite première paroi, de 37 mm.

Les données dimensionnelles quant aux diamètres données ci-dessus correspondent à des dimensions appropriées pour un ancrage du dispositif contre la muqueuse de la paroi interne de l'intestin 10 à différentes positions entre y compris le rectum. Le stent est réalisé par un maillage de fils en nitinol de 0.32mm tressés selon un angle de 30°et présente un diamètre de 34 mm en partie courante et 37mm à ses extrémités évasées, une longueur de 100mm.

Les parties proximale et distale du stent, le cas échéant les collerettes (non représentées) sont équipées respectivement chacune d'un fil lasso 6a, 6b, permettant la réduction du diamètre du stent en traction. Ces fils lassos 6a, 6b peuvent être saisis à l'aide d'une pince ou crochet dédié inséré via un endoscope.

La dite gaine 2 s'étend à l'extérieur présente une longueur couvrant la distance entre l'anastomose et le site d'ancrage en amont, soit d'au moins 10 cm.

La gaine 2 est réalisée en matériau synthétique biocompatible, radio-opaque notamment du TPU 90 AE avec 18% BaSO4, ses caractéristiques dimensionnelles sont : épaisseur de 100 µm, longueur au repos de 400 mm (hors partie interne au stent), et diamètre externe de 37 mm.

Le dit tube d'aspiration 3 constitué en matériau élastomère Pebax^{®} (ARKEMA, France) est fixé au stent par les mêmes joints de fusion 4a-4b que la gaine interne 2a aux extrémités du stent et présente une longueur couvrant au moins la distance entre l'anastomose et le site d'ancrage en amont, soit d'au moins 10 cm. Ses caractéristiques sont : longueur de 500 mm+/-2 mm ; épaisseur de 05 mm ; diamètre interne de 2 mm ; et diamètre externe de 3mm.

L'extrémité longitudinale libre extérieure dudit tube d'aspiration 3 est connectée de façon réversible à un embout de raccordement comportant un dispositif d'obturation consistant en une vanne anti-reflux, comportant un dispositif témoin de la teneur du vide dans la chambre 4.

Le stent 1, la gaine externe 2 et le tube d'aspiration injection 3 sont conditionnés dans un tube déformable plastique semi rigide 11 en matériau élastomère dénommé ci-après premier tube guide déformable 12. Ce premier tube guide 12 est une partie d'un dispositif introducteur 10 décrit ci-après.

Le stent est introduit sous forme rétractée (comprimé radialement) au niveau de l'extrémité distale amont du dit premier tube guide déformable 12, ladite gaine externe 2 et le dit tube d'aspiration 3 étant rangés repliés sur eux-mêmes en plusieurs couches superposées en partie à l'intérieur du stent et en partie l'intérieur du premier tube guide 12 de l'introducteur 10.

La fixation des extrémités avales de la gaine externe 2 et du tube d'aspiration 3 sont différentes dans les deux modes de réalisation des exemples 1 et 2, le premier tube guide 11 présentant des caractéristiques distinctives de mise en oeuvre entre les deux modes de réalisation qui seront explicitées ci-après. Le stent est retenu à l'intérieur du premier tube guide 12 par un dispositif dénommé tulipe 12a former de languettes découpées barrant le passage de l'extrémité amont ou distale du premier tube guide 12, qui empêche le stent de sortir en l'absence d'une poussée par le butée de pousseur décrite ci-après, les dites languettes étant déformables élastiquement sous l'effet d'une dite poussée permettant de d'expulser le stent en dehors du premier tube guide 12.

Le dispositif introducteur 10 comprend une poignée 14 qui est fixée à un deuxième tube rigide 13 aussi dénommé deuxième tube guide, se terminant à son extrémité distale par une partie de liaison 13a avec le premier tube guide 12. Cette partie de liaison 13a est de forme externe incurvée convexe et de plus grand diamètre que les premier tube guide déformable 12 et deuxième tube guide rigide 13 pour servir de butée.

Un moyen d'actionnement par pression ou bouton poussoir 14a sur la poignée 14 commande la translation longitudinale d'une tige de pousseur 15a se terminant par une butée de pousseur 15b d'un pousseur 15. La tige de pousseur 15a et la butée de pousseur 12b sont disposés coaxialement à l'intérieur du deuxième tube rigide 13. L'extrémité proximale de la tige de pousseur 15a coopère avec le bouton poussoir 14a sur la poignée 14. L'extrémité distale de la tige de pousseur et la butée de pousseur sont aptes à expulser le stent en dehors de l'extrémité amont (extrémité distale) du premier tube guide déformable 12 par actionnement de la tige et butée de pousseur en transaction relative à l'intérieur du premier tube guide déformable 12.

La butée du pousseur 15b est initialement positionnée juste en aval du stent à l'intérieur du premier tube guide déformable 12.

Dans les deux modes de réalisation des exemples 1 et 2 ci-après, le premier tube guide déformable 12 de l'introducteur contenant le dispositif de protection 5 compressé radialement comme décrit ci-dessus est introduit par voie abdominale dans la partie d'amont 100a de l'intestin qui a subi une section au niveau du site 101 ou l'on doit réaliser l'anastomose. En l'espèce le site d'implantation est situé à une distance de 10 cm à 20 cm en amont de l'anastomose.

A ce stade, le stent ainsi que la gaine externe 2 et le tube d'aspiration 3 sont toujours conditionnés à l'intérieur du stent, au moins en partie et à l'intérieur du premier tube guide déformable 12. Dans sa forme initiale, fermé et logé dans l'introducteur, le stent 1 présente un diamètre réduit au diamètre du tube guide déformable 11 soit notamment de 10mm.

Une fois libéré en dehors du premier tube guide déformable 12 dans la lumière de l'intestin d'amont 100a, le stent retrouve progressivement son diamètre définitif. Il peut être temporairement maintenu en place par le chirurgien, qui pince le stent à travers les parois de l'intestin. Une partie au moins de l'introducteur est ensuite retiré sans que la gaine externe 2 et le tube d'aspiration 3 ne soient dépliés et déployés en aval du stent. Ces éléments peuvent toutefois être partiellement déployés jusqu'au niveau de la zone d'anastomose (le stent étant largué entre 10 et 20 cm au-dessus de cette zone). Ceci afin de permettre la réalisation de l'anastomose avant de déplier et déployer plus complètement la gaine externe et tube d'aspiration en aval du stent. La libération du stent en dehors du premier tube guide déformable 12 dans la lumière de l'intestin, le stent retrouvant progressivement son diamètre définitif, se fait à ce stade, idéalement avant réalisation de l'anastomose.

Ledit premier tube guide déformable 12 présente un diamètre interne de 10mm, un diamètre externe de 17 mm et longueur d'au moins 70 cm, de préférence au moins 100 cm avec une graduation tous les 5 cm. Il est réalisé en matériau Pebax ^{®} permettant de limiter les frictions lors de l'introduction et retrait.

Le deuxième tube rigide 13 présente une longueur de 176 mm incluant la partie de liaison 13a incurvée convexe de diamètre protubérant de 53 mm formant butée et de 46mm de longueur. Le deuxième tube rigide 13 présente un sillon central 13b de guidage contrôlant la translation de la tige interne du pousseur.

La tige 15a dudit pousseur 15 est en acier inoxydable et présente une longueur avant la butée 15b de 487 mm. La tige 15a est une tige formée d'un fil de 1.4mm de diamètre enroulé hélicoïdalement selon un axe longitudinal virtuel XX' avec des spires jointives, le diamètre des spires jointives formant une tige déformable d'un diamètre externe de 7 mm. Une telle tige 15a peut être déformée en incurvation pour permettre de passer une courbure jusqu'à 90°.

Sur les figures 4A à 4D, on montre les différentes étapes de réalisation d'une anastomose avec une enclume 8 et une agrafeuse circulaire 9. On introduit une enclume 8 dans la partie amont 100a de l'intestin juste en amont du site de l'anastomose 101 au niveau de l'extrémité libre de la partie de l'intestin d'amont 100a.Puis, on réalise la ligature de cette extrémité libre de l'intestin avec un fil de bourse 8' autour d'un arbre 8b en arrière de la partie à section transversale circulaire 8a de l'enclume 8. Puis, on introduit dans la partie aval 100b de l'intestin l'agrafeuse 9 qui comporte un corps creux à section circulaire 9b destiné à venir recouvrir la partie circulaire 8a de l'enclume et contenant un doigt axial 9a apte à venir coopérer dans un creux axial (non représenté) dans l'arbre 8b permettant ainsi d'engager l'agrafeuse 9 pour coopérer avec l'enclume 8 afin de réaliser l'agrafage au niveau de l'anastomose 101 entre la partie d'intestin amont 100a et partie avale 100b de l'intestin avant que l'ensemble enclume 8 - agrafeuse 9 ne soit retiré de l'intestin comme montré figure 4D. Sur les figures 4B à 4D, on montre l'agrafeuse 9 en aval d'un segment d'aval d'intestin à titre schématique étant entendu que l'agrafeuse 9 est en fait introduite initialement via le rectum. On utilise notamment une agrafeuse EEA^{®} de la société COVIDIEN (France) comportant une poignée 9d et un conduit déformable 9c apte à suivre les courbures de l'intestin pour relier la poignée 9d à la partie d'agrafeuse 9b qui coopère avec l'enclume 8 pour commander l'agrafage.

Plus précisément, on réalise les étapes successives suivantes :
1) on procède à la résection de l'intestin malade grâce à une agrafeuse linéaire, ce qui entraîne la fermeture 100d et la section de l'intestin en aval 100b du segment d'intestin malade ;
2) on applique un fil de bourse 8' sur l'intestin situé en amont du segment malade avant de sectionner celui-ci ;
3) on introduit l'enclume 8 d'une agrafeuse circulaire dans l'intestin d'amont 100a et on serre et noue le fil à bourse 8' autour de son axe central 8b ;
4) on introduit le corps 9b de l'agrafeuse circulaire dans le segment d'intestin d'aval 100b (à travers l'orifice anal) jusqu'à son extrémité fermée 100d ;
5) on fait sortir l'axe central 9a de l'agrafeuse circulaire à travers les parois de l'intestin d'aval par pénétration ;
6) on solidarise l'axe central 9a de l'agrafeuse circulaire avec l'axe central 8b de l'enclume ;
7) on fait introduire les 2 axes 8a-9a réunis dans le corps de l'agrafeuse ce qui a comme conséquence d'apposer la section de l'enclume à la section du cops de l'agrafeuse ; et
8) on procède à l'agrafage et section circulaire des parois des 2 segments d'intestin 100a et 100b réunis en 101. La butée terminale 15b du pousseur est en matériau Pebax^{®}, et présente un diamètre et une épaisseur dans la direction longitudinale de la tige de 10 mm.

Dans les deux modes de réalisation des exemples 1 et 2, la gaine externe 2 et le tube d'aspiration 3 sont dépliés et déployés en aval du stent par le retrait de l'enclume après réalisation de l'agrafage.

Le retrait du dispositif de protection doit se faire idéalement après cicatrisation de la zone anastomotique dont la durée standard est de 14 jours.

Le retrait peut se faire selon 2 modes :
a) retrait par retournement au cours d'une procédure endoscopique. Dans ce cas de figure, une boucle lasso 6b située au niveau de l'extrémité distale (ou amont) du stent est saisie à l'aide d'une pince. Ceci a pour effet de réduire le diamètre du stent. Lorsque le stent est totalement rétracté radialement, il est alors possible de retourner le stent en tirant sur le fil lasso, ce qui a pour effet d'entraîner un mouvement de retournement ou inversion du stent, tout en le décollant progressivement des tissus.
b) le second mode de retrait comprend l'utilisation d'un tube de retrait. Dans ce cas, la gaine externe et le tube d'aspiration sont introduits dans un tube de retrait. Une boucle lasso 6a située au niveau de l'extrémité proximale (ou avale) du stent est saisie à l'aide d'une pince, (ce geste est guidé par l'utilisation d'un endoscope). Ceci a pour effet de réduire le diamètre du stent (par rétractation radiale). Lorsque le stent est totalement rétracté, il est alors possible de le faire glisser dans le tube de retrait. Le fait d'avancer le tube de retrait permet de décoller les tissus et de recueillir le stent de manière atraumatique. Le tube de retrait contenant le stent est ensuite retiré par voie anale.

Dans les deux modes de réalisation des exemples 1 et 2, la configuration de liaison de certains des composants de l'introducteur entre eux ou avec le dispositif de protection 1 sont différents et notamment les extrémités longitudinales avale de la gaine externe 3 et du tube d'aspiration-injection 4 sont liés à un élément du dispositif introducteur qui est différent. Les extrémités aval de la gaine externe 2 et du tube d'aspiration 3 sont fixées à l'extrémité distale du dit premier tube guide 12 dans le mode de réalisation de l'exemple 1 et à une première pièce de raccordement 11 apte à se fixer de manière réversible à la butée 15b du pousseur dans le mode de réalisation de l'exemple 2.

### EXEMPLE 1

Sur les figures 5A, 5B, 6 et 7A-7B, on a représenté un premier mode de réalisation d'un dispositif complexe selon l'invention dans lequel les extrémités avales de la gaine 2 et du tube d'aspiration 3 sont fixées en 2b-3b à proximité de l'extrémité amont ou proximale du premier tube déformable 12.

Sur la figure 5A, la gaine 2 et le tube d'aspiration 3 sont pliés à l'intérieur du stent 1 et ressortent en aval du stent au-delà du joint 4b pour aller rejoindre par l'extérieur, l'extrémité amont du premier tube guide 12, les extrémités de la gaine 2 et du tube d'aspiration 3 étant collées contre la paroi interne du premier tube guide 12 en 2b-3b à proximité de l'extrémité amont ou proximale du premier tube guide 12 .

Sur la figure 5B, on a représenté schématiquement un mode de réalisation dans lequel il y a plusieurs plis de la gaine 2 et du tube d'aspiration 3 à l'intérieur du stent 1 lesquels gaine 2 et tube d'aspiration 3 rejoignent la paroi interne du premier tube déformable 12 en étant intercalés entre la paroi externe du stent 1 et la paroi interne du premier tube guide 12. Sur la figure 5B, on a illustré de manière schématique le positionnement du stent 1 à l'intérieur du premier tube guide 12 en exagérant excessivement l'espace entre le stent 1 et la paroi interne du premier tube guide 12 pour montrer le positionnement des gaine 2 et tube d'aspiration 3 à l'extérieur du stent 1. Toutefois, en réalité, le stent 1 est en compression radiale exerçant une poussée contre la paroi interne du premier tube guide 12 avec la gaine 2 intercalée et coincée entre eux à ce stade initial.

Dans ce mode de réalisation, la butée 15b du pousseur 15 présente un diamètre en section transversale légèrement supérieur au diamètre du stent et donc apte à expulser le stent vers la sortie de l'extrémité proximale 12a du premier tube guide déformable 12 sous l'effet de la poussée en translation du pousseur 15 actionné au niveau de la poignée 14 du dispositif introducteur 10.

Sur la figure 6, on a représenté le déploiement complet de la gaine 2 et du tube d'aspiration 3 en aval du stent 1 lorsqu'après la réalisation de l'anastomose décrite ci-après en liaison avec les figures 7A-7D, on se trouve comme représenté sur la figure 7D avec le premier tube 12 retiré de l'intestin. En pratique, ce retrait se fait jusqu'à ce que l'agrafeuse sorte par l'orifice anal. A ce moment, comme représenté sur la figure 7D, on coupe avec des ciseaux 16, la gaine 2 et le tube d'aspiration 3 pour les séparer de l'ensemble complexe du premier tube 12 solidarisé à l'agrafeuse 9 par la deuxième pièce de raccordement 12b.

Dans ce mode de réalisation, comme montré sur les figures 7A-7C, l'extrémité proximale du premier tube guide déformable 12 comprend en effet une pièce dénommée deuxième pièce de raccordement 12b comprenant un filetage intérieur apte à coopérer en vissage avec un élément de filetage complémentaire 7a d'une pièce dénommée adaptateur 7, cette dernière étant apte à être fixée par exemple par collage réversible avec la face frontale 8c de l'enclume 8.

Dans ce mode de réalisation, la deuxième pièce de raccordement 12b sert initialement à recevoir l'introducteur 10 comme représenté sur la figure 2 par vissage de l'élément de filetage complémentaire 13b à l'extrémité distale du deuxième tube guide rigide 13 pour arriver à la configuration des figures 2A et 5A dans lequel la butée 15b arrive à proximité de l'extrémité avale du stent 1 à l'intérieur du premier tube guide 12. A ce moment, on actionne la tige de pousseur 15a par translation relative du deuxième tube guide rigide 13 par rapport à la poignée 14 de par une échancrure 13c le long du deuxième tube rigide 13. Cette translation est commandée par actionnement du poussoir 14a, pour expulser le stent 1 en dehors du premier tube guide 12 comme montré sur la figure 2B. A ce stade, la deuxième pièce de raccordement 12b se situe sensiblement au niveau de l'extrémité libre de la partie amont 100a de l'intestin et est donc en position pour recevoir l'enclume 8 via l'intercalation d'un adaptateur 7 comme montré sur les figures 7A à 7C. Ensuite, on réalise l'anastomose avec l'agrafeuse 9 comme décrit en référence aux figures 4A à 4C. Puis, on retire l'ensemble des premier tube guide 12 - enclume 8 -agrafeuse 9 depuis l'extérieur du patient au niveau de l'orifice anal pour arriver à la configuration illustrée par les figures 6 et 7D. Sur la figure 7D, on montre l'agrafeuse en aval d'un segment d'aval d'intestin 100b à titre schématique étant entendu que l'agrafeuse 9 est en fait retirée de l'intestin via le rectum.

Dans ce mode de réalisation, de préférence, la gaine 2 et le tube d'aspiration 3 ne sont pas fixés directement à l'extrémité du tube déformable 12 en 2b et 3b mais par l'intermédiaire d'un lien comme un fil de suture (non représenté).

### EXEMPLE 2 :

Sur les figures 8A-8B, 9A-9B, 10A-10C et 11A-11B, on a illustré les caractéristiques distinctives d'un second mode de réalisation d'un dispositif complexe selon l'invention.

Dans ce deuxième mode de réalisation, les extrémités avales de la gaine 2 et du tube d'aspiration 3 sont fixées à une première pièce de raccordement 11 positionnée juste à l'extérieur de l'extrémité aval du stent 1.

Les figures 8A et 8B illustrent 2 variantes de disposition de la gaine 2 et du tube d'aspiration 3 à l'intérieur du stent 1. Dans les 2 cas, la gaine 2 et le tube d'aspiration 3 sont disposés initialement à l'intérieur du stent. Dans la première variante préférée de la figure 8A, le tube d'aspiration 3 et la gaine 2 sont simplement fixés au niveau d'une partie tubulaire 11a à l'extrémité distale de la première pièce de raccordement 11 et pliés à l'intérieure du stent 1, le tube d'aspiration 3 n'étant pas enroulé sur la partie tubulaire 11a comme dans la deuxième variante de la figure 8B. Les différents stades de mise en oeuvre du dispositif complexe selon l'invention selon ce deuxième mode de réalisation sont illustrés par rapport à cette deuxième variante de la figure 8B mais peuvent être transposés à la variante de la figure 8A. La partie tubulaire 11a de la pièce de raccordement 11 rentre initialement à l'intérieur du stent 1 à son extrémité avale et la partie de plus grand diamètre 11b sert d'appui à la butée 15b de la tige du pousseur pour la phase d'introduction puis sert à la fixation réversible de la pièce de raccordement 11 contre la face avant frontale 8c de l'enclume 8 pour la phase d'agrafage et réalisation de l'anastomose.

Dans ce mode de réalisation, le dispositif complexe selon l'invention comprenant l'ensemble du dispositif de protection 5 et de la première pièce de raccordement 11, lequel ensemble est indépendant du premier tube guide 12. Ainsi, le premier tube guide 12 peut être fixé de manière irréversible au niveau de la pièce de liaison 13a à l'extrémité distale du deuxième tube guide rigide 13 de l'introducteur 10 comme montré sur la figure 9A.

Sur les figures 9A et 9B, on montre qu'initialement la butée 15b à l'extrémité distale de la tige 15a du pousseur vient s'appliquer contre la face proximale plate 11b de la première pièce de raccordement 11 pour expulser le stent 1 en dehors du premier tube guide 12 par translation de la tige du pousseur 15. Ensuite, comme montré figure 10A, on retire l'introducteur 10 pour le sortir de la partie d'intestin amont 100a pour laisser la première pièce de raccordement 11 au niveau du site de l'anastomose 101. Il est avantageux que la butée 15a soit collée de manière réversible à la première pièce de raccordement 11 pour permettre de déployer partiellement la gaine 2 et le tube d'aspiration 3 lors du retrait partiel préliminaire de l'introducteur 10 jusqu'à ce que la pièce 11 arrive au site de l'anastomose comme montré sur la figure 10B. A ce stade, on fixe de manière réversible l'enclume 8 contre la première pièce de raccordement 11 par exemple par collage réversible. Puis, on réalise l'anastomose comme dans l'exemple 1 et comme illustré sur les figures 4A à 4C jusqu'à arriver à la situation montrée figures 11A et 11B dans laquelle on retire l'ensemble solidaire des première pièce de raccordement 11-enclume 8-agrafeuse 9 pour déployer la gaine 2 et le tube 3 totalement en aval du stent 1 et en aval de l'anastomose 101 jusqu'à l'orifice anal au niveau duquel on sépare la gaine 2 et le tube d'aspiration 3 de la première pièce de raccordement 11avec les ciseaux 16. Sur les figures 11A-11B, on montre l'agrafeuse 9 en aval d'un segment d'aval d'intestin 100b à titre schématique étant entendu que l'agrafeuse 9 est en fait introduite et retirée de l'intestin via le rectum la poignée 9d restant à l'extérieur du rectum.

Dans un exemple d'une version simplifiée (non représentée), la gaine souple 2 et deux tubes d'aspiration 2 sont initialement disposés dans le dit premier tube 12 mais entièrement à l'extérieur du stent, en aval du stent le dit stent étant maintenu en compression radiale à l'intérieur du dit premier tube guide, et les deux tubes d'aspirations 2 étant disposés de manière à pouvoir s'étendre de manière diamétralement opposée par rapport audit stent et à l'extérieur de la gaine.

## Revendications

1. Dispositif chirurgical complexe constitué d'un ensemble prêt à être utilisé pour la réalisation d'une anastomose (101) dans l'intestin avec une enclume (8) et une agrafeuse circulaire (9) et la protection de ladite anastomose dans l'intestin comprenant :
(a) un dispositif de protection (5) comprenant un élément d'ancrage constitué d'au moins un stent (1) apte à s'ancrer temporairement sur la paroi interne de l'intestin en amont (100a) de l'anastomose et une gaine souple (2) dont l'extrémité amont au moins est fixée audit stent, ladite gaine étant apte à s'étendre en aval dudit élément d'ancrage et en aval de la dite anastomose pour permettre la protection de la dite anastomose, et
(b) au moins un premier tube dénommé premier tube guide (12), le dit stent (1) étant maintenu en compression radiale à l'intérieur du dit premier tube guide (12), ledit premier tube guide (12) étant une partie d'un tube guide d'un dispositif introducteur (10) ou étant apte à être fixé de manière réversible à un dispositif introducteur (10), le dit premier tube guide étant déformable en incurvation par rapport à son axe longitudinal (XX'),
**caractérisé en ce que** ladite gaine souple (2) est rangée, à l'intérieur du dit premier tube guide (12), l'extrémité avale (2b) de la dite gaine étant liée audit premier tube guide (12) ou à une première pièce de raccordement (11) indépendante dudit premier tube guide (12), ladite gaine (2) étant apte à être déployée en aval dudit stent par le retrait de l'extrémité proximale dudit premier tube guide (12) ou respectivement à ladite première pièce de raccordement (11) à laquelle elle est fixée.

2. Dispositif chirurgical complexe selon la revendication 1, dans lequel le dispositif de protection (5) comprend au moins un tube souple dénommé tube d'aspiration (3), de préférence deux tubes d'aspiration, chaque dit tube d'aspiration étant apte à s'étendre à l'extérieur dudit stent en aval de celui-ci, une extrémité ouverte dudit tube d'aspiration débouchant à l'intérieur du stent dans une chambre de dépression (4) délimitée entre la paroi interne du stent et un film interne recouvrant la paroi interne du stent de préférence ledit film constituant une prolongation de la dite gaine souple (2), **caractérisé en ce que** chaque dit tube d'aspiration (3) est rangé, de préférence plié ou enroulé hélicoïdalement, à l'intérieur du dit premier tube guide (12), l'extrémité avale (3b) de chaque dit tube d'aspiration (3) étant liée audit premier tube guide (12) ou à une dite première pièce de raccordement (11), chaque dit tube d'aspiration (3) étant apte à être déployé en aval dudit stent par le retrait de la dite enclume fixée de manière réversible à l'extrémité proximale dudit premier tube guide (12) ou respectivement à ladite première pièce de raccordement (11).

3. Dispositif chirurgical complexe selon l'une des revendications 1 et 2 **caractérisé en ce que** l'extrémité avale (2) de la dite gaine (2) et l'extrémité avale du (ou des) dit(s) tube(s) d'aspiration (3) sont reliées à l'extrémité distale du dit premier tube guide, l'extrémité proximale dudit premier tube guide comprenant une deuxième pièce de raccordement (12b) apte à se fixer de manière réversible à l'extrémité distale d'un deuxième tube guide rigide (13) d'un dispositif introducteur (10) pour l'introduction du dispositif de protection en amont du site de l'anastomose et apte à se fixer ensuite de manière réversible à une dite enclume (8) pour la réalisation de l'anastomose.

4. Dispositif chirurgical complexe selon la revendication 3, **caractérisé en ce que** l'extrémité avale (2) de la dite gaine (2) et l'extrémité avale du (ou des) dit(s) tube(s) d'aspiration (3) sont reliées à l'extrémité distale du dit premier tube guide par collage (2b, 3b) ou par un fil.

5. Dispositif chirurgical complexe selon l'une des revendications 3 ou 4, **caractérisé en ce que** la dite deuxième pièce de raccordement (12b) est fixée ou apte à se fixer de manière réversible à un adaptateur (7), ledit adaptateur étant lui-même apte à se fixer de manière réversible à une dite enclume (8) d'agrafeuse.

6. Dispositif chirurgical complexe selon l'une des revendications 3 à 5, **caractérisé en ce que** la dite deuxième pièce de raccordement (12b) fixée ou est apte à se fixer de manière réversible par vissage, collage, clampage ou liaison magnétique, à un adaptateur (7) lui-même apte à se fixer de manière réversible à une dite enclume (8), par collage, vissage, clampage ou liaison magnétique.

7. Dispositif chirurgical complexe selon l'une des revendications 3 à 6 **caractérisé en ce que** la dite deuxième pièce de raccordement (12b) comprend un élément de filetage apte à coopérer par vissage avec :
- un premier élément de filetage complémentaire (13b) situé à l'extrémité distale du dit deuxième tube guide rigide (13), puis
- un deuxième élément de filetage complémentaire (7a) situé à l'extrémité distale d'un dit adaptateur (7).

8. Dispositif chirurgical complexe selon l'une des revendications 1 ou 2 **caractérisé en ce que** l'extrémité avale de la dite gaine et de préférence l'extrémité avale du (ou des) dit(s) tube(s) d'aspiration (3), est (ou sont) liée(s) à une dite première pièce de raccordement (11) dont une extrémité proximale (11b) au moins est disposée à l'extérieure de l'extrémité avale dudit stent.

9. Dispositif chirurgical complexe selon la revendication 8 **caractérisé en ce que** l'extrémité avale de la dite gaine souple (2) et de préférence l'extrémité avale du (ou des) dit(s) tube(s) d'aspiration (3), est (ou sont) liée(s) à une partie distale (11a) d'une dite première pièce de raccordement (11) indépendante dudit premier tube guide (12), de préférence une partie distale tubulaire (11a) rangée ou apte à se ranger à l'intérieur de l'extrémité avale dudit stent, et une partie proximale (11b) de la dite première pièce de raccordement (11) disposée à l'extérieur et à l'extrémité avale dudit stent présentant de préférence une face proximale plate de plus grand diamètre que ladite partie distale tubulaire (11a).

10. Dispositif chirurgical complexe selon la revendication 8 ou 9, **caractérisé en ce que** l'extrémité avale de la dite gaine souple (2) et l'extrémité avale du (ou des) dit(s) tube(s) d'aspiration (3) sont liées à une dite première pièce de raccordement (11) par collage ou par un fil.

11. Dispositif chirurgical complexe selon la revendication 9 ou 10, **caractérisé en ce que** la dite première pièce de raccordement (11) est apte à se fixer de manière réversible à une butée (15b) d'une tige (15a) de pousseur à l'intérieur dudit premier tube guide lors de l'introduction du dispositif de protection en amont du site de l'anastomose, de préférence par collage, vissage, clampage ou liaison magnétique, de préférence encore par collage.

12. Dispositif chirurgical complexe selon l'une des revendications 8 à 11, **caractérisé en ce que** la dite première pièce de raccordement (11) est apte à se fixer de manière réversible directement à une dite enclume (8) par collage, vissage, clampage ou liaison magnétique, de préférence par collage.

13. Dispositif chirurgical complexe selon l'une des revendications 1 à 12, **caractérisé en ce que** l'extrémité distale du dit premier tube guide (12) est fermée par un élément de retenue flexible (12a) apte à retenir le dit dispositif de protection (5) à l'intérieur du dit premier tube guide en absence de poussée à l'intérieur du dit premier tube guide par une butée (15a) de tige de pousseur, le dit élément de retenue étant apte à se déformer élastiquement et autoriser la sortie dudit dispositif de protection (5) sous l'effet de la dite poussée par la dite butée du pousseur.

14. Dispositif chirurgical complexe selon l'une des revendications 1 à 13, **caractérisé en ce qu'**il comprend un dit dispositif de protection (5) et un introducteur (10) apte à permettre l'implantation de l'élément d'ancrage (1) du dispositif de protection (5.) en amont du site de l'anastomose, ledit introducteur (10) comprenant une poignée (14) fixée et/ou apte à coopérer avec les deux parties suivantes de l'introducteur :
(b1) un dit premier tube guide (12) à l'intérieur duquel ledit stent est maintenu en compression radiale à proximité de l'extrémité distale dudit premier tube guide, l'extrémité proximale dudit premier tube guide étant fixée à un deuxième tube guide (13) rigide solidaire de la dite poignée, et
(b2) un pousseur (15) comprenant une tige de pousseur (15a) déformable en incurvation par rapport à son axe longitudinal (XX') et une dite butée de pousseur (15b) à l'extrémité distale de la tige de pousseur, s'étendant depuis la poignée à l'intérieure dudit deuxième tube guide rigide (13) et dudit premier tube guide (12), l'extrémité proximale de ladite tige de pousseur (15a) étant apte à coopérer avec ladite poignée en commandant manuellement une translation relative de ladite tige de pousseur par rapport au premier tube guide.

15. Dispositif chirurgical complexe selon la revendication 14, **caractérisé en ce que** la tige de pousseur (15a) est une tige spiralée formée d'un fil en acier enroulé hélicoïdalement selon un axe longitudinal virtuel (XX') avec des spires jointives coaxiales de même diamètre, le diamètre des spires jointives formant une tige déformable apte à être déformée en incurvation par rapport audit axe longitudinal pour permettre d'adopter une courbure jusqu'à former un coude à 90°.

## Patentansprüche

1. Komplexe chirurgische Vorrichtung für eine gebrauchsfertige Anordnung zur Durchführung einer Anastomose (101) im Darm mit einem Amboss (8) und einem kreisförmigen Klammerapparat (9) und Schutz der Anastomose in dem Darm, die aufweist:
(a) eine Schutzvorrichtung (5), die ein Ankerelement, das aus wenigstens einem Stent (1) besteht, der fähig ist, laufaufwärtig (100a) von der Anastomose vorübergehend an der Innenwand des Darms verankert zu werden, und eine flexible Umhüllung (2), von der wenigstens das laufaufwärtige Ende an dem Stent fixiert ist, aufweist, wobei die Umhüllung fähig ist, sich laufabwärtig von dem Ankerelement und laufabwärtig von der Anastomose zu erstrecken, um zu ermöglichen, dass die Anastomose geschützt wird, und
(b) wenigstens ein erstes Rohr, das als ein erstes Führungsrohr (12) bezeichnet wird, wobei der Stent (1) im Inneren des ersten Führungsrohrs (12) radial zusammengedrückt gehalten wird, wobei das erste Führungsrohr (12) ein Teil eines Führungsrohrs einer Einführungsvorrichtung (10) ist oder reversibel an einer Einführungsvorrichtung (10) anbringbar ist, wobei das erste Führungsrohr in der Krümmung in Bezug auf seine Längsachse (XX') verformbar ist,
**dadurch gekennzeichnet, dass** die flexible Umhüllung (2) im Inneren des ersten Führungsrohrs (12) gelagert wird, wobei das strömungsabwärtige Ende (2b) der Umhüllung mit dem ersten Führungsrohr (12) oder mit einem ersten Verbindungsstück (11), das von dem ersten Führungsrohr (12) unabhängig ist, verbunden wird, wobei die Umhüllung (2) fähig ist, laufabwärtig von dem Stent eingesetzt zu werden, indem das proximale Ende des ersten Führungsrohrs (12) oder jeweils das erste Verbindungsstück (11), an dem sie fixiert ist, zurückgezogen wird.

2. Komplexe chirurgische Vorrichtung nach Anspruch 1, wobei die Schutzvorrichtung (5) wenigstens ein flexibles Rohr, das als Saugrohr (3) bezeichnet wird, vorzugsweise zwei Saugrohre, aufweist, wobei jedes Saugrohr fähig ist, sich außerhalb des Stents laufabwärtig davon zu erstrecken, und wobei ein offenes Ende des Saugrohrs sich im Inneren des Stents in eine Vakuumkammer (4) öffnet, die zwischen der Innenwand des Stents und einer inneren Dünnschicht, welche die Innenwand des Stents bedeckt, definiert ist, wobei die Dünnschicht vorzugsweise eine Verlängerung der flexiblen Umhüllung (2) bildet, **dadurch gekennzeichnet, dass** jedes Saugrohr (3) vorzugsweise gefaltet oder spiralförmig gewickelt im Inneren des ersten Führungsrohrs (12) angeordnet ist, wobei das laufabwärtige Ende (3b) jedes Saugrohrs (3) mit dem ersten Führungsrohr (12) oder dem ersten Verbindungsstück (11) verbunden ist, wobei jedes Saugrohr (3) geeignet ist, laufabwärtig von dem Stent eingesetzt zu werden, indem der Amboss, der reversibel an dem proximalen Ende des ersten Führungsrohrs (12) oder jeweils an dem ersten Verbindungsstück (11) fixiert ist, zurückgezogen wird.

3. Komplexe chirurgische Vorrichtung nach einem der Ansprüche 1 und 2, **dadurch gekennzeichnet, dass** das laufabwärtige Ende (2) der Umhüllung (2) und das laufabwärtige Ende des/der Saugrohrs/e (3) mit dem distalen Ende des ersten Verbindungsrohrs verbunden ist/sind, wobei das proximale Ende des ersten Führungsrohrs ein zweites Verbindungsstück (12b) aufweist, das fähig ist, in einer reversiblen Weise an dem distalen Ende eines zweiten starren Führungsrohrs (13) einer Einführungsvorrichtung (10) für die Einführung der Schutzvorrichtung laufaufwärtig von der Anastomosestelle fixiert zu werden, und fähig ist, dann in einer reversiblen Weise an dem Amboss (8) fixiert zu werden, um die Anastomose auszuführen.

4. Komplexe chirurgische Vorrichtung nach Anspruch 3, **dadurch gekennzeichnet, dass** das laufabwärtige Ende (2) der Umhüllung (2) und das laufabwärtige Ende des/der Saugrohrs/e (3) durch Kleben (2b, 3b) oder durch ein Gewinde mit dem distalen Ende des ersten Führungsrohrs verbunden sind.

5. Komplexe chirurgische Vorrichtung nach Anspruch 3 oder 4, **dadurch gekennzeichnet, dass** das zweite Verbindungsstück (12b) in einer reversiblen Weise an einem Adapter (7) fixiert ist oder fähig ist, daran fixiert zu werden, wobei der Adapter selbst fähig ist, in einer reversiblen Weise an dem Amboss (8) des Klammerapparats fixiert zu werden.

6. Komplexe chirurgische Vorrichtung nach einem der Ansprüche 3 bis 5, **dadurch gekennzeichnet, dass** das zweite Verbindungsstück (12b) in einer reversiblen Weise an einem Adapter (7), der selbst fähig ist, in einer reversiblen Weise durch Leimen, Schrauben, Klammern oder eine magnetische Verbindung an dem Amboss (8) fixiert zu werden, durch Schrauben, Leimen, Klammern oder eine magnetische Verbindung fixiert wird oder fähig ist, daran fixiert zu werden.

7. Komplexe chirurgische Vorrichtung nach einem der Ansprüche 3 bis 6, **dadurch gekennzeichnet, dass** das zweite Verbindungsstück (12b) ein Gewindeelement aufweist, das fähig ist, durch Schrauben zusammenzuwirken mit:
- einem ersten komplementären Gewindeelement (13b), das an dem distalen Ende des zweiten starren Führungsrohrs (13) angeordnet ist, dann
- einem zweiten komplementären Gewindeelement (7a), das an dem distalen Ende eines der Adapter (7) angeordnet ist.

8. Komplexe chirurgische Vorrichtung nach Anspruch 1 oder 2, **dadurch gekennzeichnet, dass** das laufabwärtige Ende der Umhüllung und vorzugsweise das laufabwärtige Ende des/der Saugrohrs/e (3) mit dem ersten Verbindungsstück (11) verbunden ist/sind, von dem wenigstens ein proximales Ende (11b) außerhalb des laufabwärtigen Endes des Stents angeordnet ist.

9. Komplexe chirurgische Vorrichtung nach Anspruch 8, **dadurch gekennzeichnet, dass** das laufabwärtige Ende der flexiblen Umhüllung (2) und vorzugsweise das laufabwärtige Ende des/der Saugrohrs/e (3) mit einem distalen Abschnitt (11a) des ersten Verbindungsstücks (11), das unabhängig von dem ersten Führungsrohr (12) ist, verbunden ist/sind, wobei ein rohrförmiger distaler Abschnitt (11a) vorzugsweise im Inneren des laufabwärtigen Endes des Stents angeordnet wird oder fähig ist, dort angeordnet zu werden, und ein proximaler Abschnitt (111b) des ersten Verbindungsstücks (11) außerhalb und an dem laufabwärtigen Ende des Stents, der vorzugsweise eine flache proximale Seite mit einem größeren Durchmesser als der rohrförmige distale Abschnitt (11a) hat, angeordnet wird.

10. Komplexe chirurgische Vorrichtung nach Anspruch 8 oder 9, **dadurch gekennzeichnet, dass** das laufabwärtige Ende der flexiblen Umhüllung (2) und das laufabwärtige Ende des/der Saugrohrs/e (3) durch Leimen oder durch ein Gewinde an das erste Verbindungsstück (11) geklebt sind.

11. Komplexe chirurgische Vorrichtung nach Anspruch 9 oder 10, **dadurch gekennzeichnet, dass** das erste Verbindungsstück (11) fähig ist, vorzugsweise durch Leimen, Schrauben, Klammern oder eine magnetische Verbindung, wiederum vorzugsweise durch Leimen, reversibel an einem Anschlag (15b) einer Schubstange (15a) im Inneren des ersten Führungsrohrs fixiert zu werden, wenn die Schutzvorrichtung laufaufwärtig von der Anastomosestelle eingeführt wird.

12. Komplexe chirurgische Vorrichtung nach einem der Ansprüche 8 bis 11, **dadurch gekennzeichnet, dass** das erste Verbindungsstück (11) geeignet ist, durch Leimen, Schrauben, Klammern oder eine magnetische Verbindung, vorzugsweise durch Leimen, reversibel direkt an einem der Ambosse (8) fixiert zu werden.

13. Komplexe chirurgische Vorrichtung nach einem der Ansprüche 1 bis 12, **dadurch gekennzeichnet, dass** das distale Ende des ersten Führungsrohrs (12) durch ein flexibles Halteelement (12a) geschlossen wird, welches fähig ist, die Schutzvorrichtung (5) im Inneren des ersten Führungsrohrs festzuhalten, wenn kein Schub durch einen Schubstangenanschlag (15a) im Inneren des ersten Führungsrohrs vorhanden ist, wobei das Halteelement fähig ist, sich elastisch zu verformen, und den Austritt der Schutzvorrichtung (5) unter der Wirkung des Schubs durch den Schubstangenanschlag zu ermöglichen.

14. Komplexe chirurgische Vorrichtung nach einem der Ansprüche 1 bis 13, **dadurch gekennzeichnet, dass** sie eine genannte Schutzvorrichtung (5) und eine Einführungsvorrichtung (10) aufweist, die fähig ist, zu ermöglichen, dass das Ankerelement (1) der Schutzvorrichtung (5) laufaufwärtig von der Anastomosestelle implantiert wird, wobei die Einführungsvorrichtung (10) einen Griff (14) aufweist, der an den nächsten zwei Teilen der Einführungsvorrichtung fixiert ist und/oder fähig ist, mit diesen zusammenzuwirken:
(b1) einem ersten Führungsrohr (12), innerhalb dem der Stent in der Nähe des distalen Endes des ersten Führungsrohrs radial zusammengedrückt gehalten wird, wobei das proximale Ende des ersten Führungsrohrs an einem zweiten starren Führungsrohr (13), das mit dem Griff integral ist, fixiert wird, und
(b2) einem Schieber (15), der eine Schubstange (15a), deren Krümmung in Bezug auf ihre Längsachse (XX') verformbar ist, und einen Schieberanschlag (15b) an dem distalen Ende der Schubstange aufweist, der sich von dem Griff im Inneren des zweiten starren Führungsrohrs (13) und dem ersten Führungsrohr (12) erstreckt, wobei das proximale Ende der Schubstange (15a) geeignet ist, mit dem Griff zusammenzuwirken, indem eine relative Verschiebung der Schubstange in Bezug auf das erste Führungsrohr manuell gesteuert wird.

15. Komplexe chirurgische Vorrichtung nach Anspruch 14, **dadurch gekennzeichnet, dass** die Schubstange (15a) eine Spiralstange ist, die aus einem Stahldraht ausgebildet ist, der mit koaxialen zusammenhängenden Windungen mit dem gleichen Durchmesser spiralförmig entlang einer virtuellen Längsachse (XX') gewickelt ist, wobei der Durchmesser der zusammenhängenden Wicklungen eine verformbare Stange ausbildet, deren Krümmung fähig ist, in Bezug auf die Längsachse verformt zu werden, um zu ermöglichen, dass sie eine 90°-Biegung annimmt.

## Claims

1. A complex surgical device consisting of a ready-to-use assembly for performing an anastomosis (101) in the intestine with an anvil (8) and a circular stapler (9) and protecting said anastomosis in the intestine comprising:
(a) a protective device (5) comprising an anchor element consisting of at least one stent (1) capable of being temporarily anchored to the inner wall of the intestine upstream (100a) of the anastomosis and a flexible sheath (2), at least the upstream end of which is fixed to said stent, said sheath being capable of extending downstream of said anchor element and downstream of said anastomosis in order to allow said anastomosis to be protected, and
(b) at least a first tube, called first guide tube (12), said stent (1) being held in radial compression inside said first guide tube (12), said first guide tube (12) being part of a guide tube of an introducer device (10) or being reversibly attachable to an introducer device (10), said first guide tube being deformable in curvature with respect to its longitudinal axis (XX'),
**characterized in that** said flexible sheath (2) is stored, inside said first guide tube (12), the downstream end (2b) of said sheath being connected to said first guide tube (12) or to a first connection piece (11) independent of said first guide tube (12), said sheath (2) being able to be deployed downstream of said stent by withdrawing the proximal end of said first guide tube (12) or respectively to said first connection piece (11) to which it is fixed.

2. The complex surgical device according to claim 1, wherein the protective device (5) comprises at least one flexible tube, called suction tube (3), preferably two suction tubes, each said suction tube being capable of extending outside said stent downstream thereof, an open end of said suction tube opening inside the stent into a vacuum chamber (4) defined between the inner wall of the stent and an inner film covering the inner wall of the stent, preferably said film constituting an extension of said flexible sheath (2), **characterized in that** each said suction tube (3) is arranged, preferably folded or wound helically, inside said first guide tube (12), the downstream end (3b) of each said suction tube (3) being connected to said first guide tube (12) or to a said first connecting piece (11), each said suction tube (3) being adapted to be deployed downstream of said stent by the withdrawal of said anvil reversibly fixed to the proximal end of said first guide tube (12) or respectively to said first connecting piece (11).

3. The complex surgical device according to any one of claims 1 and 2, **characterized in that** the downstream end (2) of said sheath (2) and the downstream end of said suction tube(s) (3) are connected to the distal end of said first guide tube, the proximal end of said first guide tube comprising a second connecting piece (12b) capable of being fixed in a reversible manner to the distal end of a second rigid guide tube (13) of an introducer device (10) for the introduction of the protective device upstream of the anastomosis site and capable of then being fixed in a reversible mannerto a said anvil (8) for carrying out the anastomosis.

4. The complex surgical device according to claim 3, **characterized in that** the downstream end (2) of said sheath (2) and the downstream end of said suction tube(s) (3) are connected to the distal end of said first guide tube by bonding (2b, 3b) or by a thread.

5. The complex surgical device according to any one of claim 3 or 4, **characterized in that** said second connecting piece (12b) is fixed or capable of being fixed in a reversible manner to an adapter (7), said adapter being itself capable of being fixed in a reversible manner to a said stapler anvil (8).

6. The complex surgical device according to any one of claims 3 to 5, **characterized in that** said second connecting piece (12b) is fixed or is capable of being fixed in a reversible manner by screwing, gluing, clamping or magnetic connection, to an adapter (7) which is itself capable of being fixed in a reversible manner to a said anvil (8), by gluing, screwing, clamping or magnetic connection.

7. The complex surgical device according to any one of claims 3 to 6, **characterized in that** said second connecting piece (12b) comprises a threaded element capable of cooperating by screwing with:
- a first complementary threaded element (13b) located at the distal end of said second rigid guide tube (13), then
- a second complementary threaded element (7a) located at the distal end of a said adapter (7).

8. The complex surgical device according to any one of claim 1 or 2, **characterized in that** the downstream end of said sheath and preferably the downstream end of said suction tube(s) (3), is (or are) connected to a said first connecting piece (11) of which at least one proximal end (11b) is disposed outside the downstream end of said stent.

9. The complex surgical device according to claim 8, **characterized in that** the downstream end of said flexible sheath (2) and preferably the downstream end of said suction tube(s) (3), is (or are) connected to a distal portion (11a) of a said first connecting piece (11) independent of said first guide tube (12), preferably a tubular distal portion (11a) arranged or capable of being arranged inside the downstream end of said stent, and a proximal portion (11b) of said first connecting piece (11) arranged outside and at the downstream end of said stent preferably having a flat proximal face of larger diameter than said tubular distal portion (11a).

10. The complex surgical device according to claim 8 or 9, **characterized in that** the downstream end of said flexible sheath (2) and the downstream end of said suction tube(s) (3) are bonded to a said first connecting piece (11) by gluing or by a thread.

11. The complex surgical device according to claim 9 or 10, **characterized in that** said first connecting piece (11) is capable of being reversibly fixed to a stop (15b) of a pusher rod (15a) inside said first guide tube when the protective device is introduced upstream of the anastomosis site, preferably by gluing, screwing, clamping or magnetic bonding, again preferably by gluing.

12. The complex surgical device according to any one of claims 8 to 11, **characterized in that** said first connecting piece (11) is adapted to be reversibly fixed directly to a said anvil (8) by gluing, screwing, clamping or magnetic bonding, preferably by gluing.

13. The complex surgical device according to any one of claims 1 to 12, **characterized in that** the distal end of said first guide tube (12) is closed by a flexible retaining element (12a) capable of retaining said protective device (5) inside said first guide tube in the absence of a thrust inside said first guide tube by a pusher rod stop (15a), said retaining element being capable of deforming elastically and allowing the exit of said protective device (5) under the effect of said thrust by said pusher rod stop.

14. The complex surgical device according to any one of claims 1 to 13, **characterized in that** it comprises a said protective device (5) and an introducer (10) capable of allowing the anchor element (1) of the protective device (5) to be implanted upstream of the anastomosis site, said introducer (10) comprising a handle (14) fixed and/or capable of cooperating with the next two parts of the introducer:
(b1) a said first guide tube (12) within which said stent is held in radial compression near the distal end of said first guide tube, the proximal end of said first guide tube being fixed to a second rigid guide tube (13) integral with said handle, and
(b2) a pusher (15) comprising a pusher rod (15a) deformable in curvature with respect to its longitudinal axis (XX') and a said pusher stop (15b) at the distal end of the pusher rod, extending from the handle inside said second rigid guide tube (13) and said first guide tube (12), the proximal end of said pusher rod (15a) being adapted to cooperate with said handle by manually controlling a relative translation of said pusher rod with respect to the first guide tube.

15. The complex surgical device according to claim 14, **characterized in that** the pusher rod (15a) is a spiral rod formed of a steel wire wound helically along a virtual longitudinal axis (XX') with coaxial contiguous turns of the same diameter, the diameter of the contiguous turns forming a deformable rod capable of being deformed in curvature with respect to said longitudinal axis to allow it to adopt a curvature to form a 90° bend.
